# FASCICULE DE BREVET EUROPEEN

(11) **EP 3 152 309 B1**
(45) Date de publication et mention de la délivrance du brevet: **02.09.2020**
(21) Numéro de dépôt: 15774647.0
(22) Date de dépôt: 03.06.2015
(51) Int. Cl.: C12N 15/82

(54) **UTILISATION DE MICROPEPTIDES POUR FAVORISER LA CROISSANCE DES PLANTES**
VERWENDUNG VON MIKROPEPTIDEN ZUR STIMULIERUNG DES PFLANZENWACHSTUMS
USE OF MICROPEPTIDES IN ORDER TO STIMULATE PLANT GROWTH

(30) Priorité: 03.06.2014 FR 1455045; 03.06.2014 FR 1455046; 24.03.2015 FR 1552469
(43) Date de publication de la demande: 12.04.2017
(73) Titulaire: Université Toulouse III-Paul Sabatier, 31062 Toulouse Cedex 9 (FR); Centre National de la Recherche Scientifique, 75794 Paris Cedex 16 (FR)
(72) Inventeur: COMBIER, Jean-Philippe, 31320 Castanet Tolosan (FR); LAURESSERGUES, Dominique, 31000 Toulouse (FR); BECARD, Guillaume, 31450 Odars (FR)
(74) Mandataire: Grosset-Fournier, Chantal Catherine
(86) Numéro de dépôt international: PCT/FR2015/051472
(87) Numéro de publication internationale: WO 2015/185861

(56) Documents cités:
- WO-A1-2015/063431
- GUO H S ET AL: "MicroRNA directs mRNA cleavage of the transcription factor NAC1 to downregulate auxin signals for arabidopsis lateral root development", THE PLANT CELL, AMERICAN SOCIETY OF PLANT BIOLOGISTS, US, vol. 17, no. 5, 1 mai 2005 (2005-05-01), pages 1376-1386, XP002734737, ISSN: 1040-4651, DOI: 10.1105/TPC.105.030841 [extrait le 2005-04-13]
- LAUFS P ET AL: "MicroRNA regulation of the CUC genes is required for boundary size control in Arabidopsis meristems", DEVELOPMENT, THE COMPANY OF BIOLOGISTS LTD, GB, vol. 131, no. 17, 1 septembre 2004 (2004-09-01), pages 4311-4322, XP002394624, ISSN: 0950-1991, DOI: 10.1242/DEV.01320
- YAO X ET AL: "Two types of cis-acting elements control the abaxial epidermis-specific transcription of the MIR165a and MIR166a genes", FEBS LETTERS, ELSEVIER, AMSTERDAM, NL, vol. 583, no. 22, 19 novembre 2009 (2009-11-19), pages 3711-3717, XP026782232, ISSN: 0014-5793, DOI: 10.1016/J.FEBSLET.2009.10.076 [extrait le 2009-10-29]
- ZHOU GONG-KE ET AL: "Overexpression of miR165 affects apical meristem formation, organ polarity establishment and vascular development in Arabidopsis", PLANT AND CELL PHYSIOLOGY, vol. 48, no. 3, mars 2007 (2007-03), pages 391-404, XP002750170, ISSN: 0032-0781
- Y. MAO ET AL: "microRNA319a-Targeted Brassica rapa ssp. pekinensis TCP Genes Modulate Head Shape in Chinese Cabbage by Differential Cell Division Arrest in Leaf Regions", PLANT PHYSIOLOGY, vol. 164, no. 2, 18 décembre 2013 (2013-12-18), pages 710-720, XP055164109, ISSN: 0032-0889, DOI: 10.1104/pp.113.228007
- CHUNHUA YANG ET AL: "Overexpression of microRNA319 impacts leaf morphogenesis and leads to enhanced cold tolerance in rice ( O ryza sativa L.)", PLANT, CELL & ENVIRONMENT, vol. 36, no. 12, 30 mai 2013 (2013-05-30), pages 2207-2218, XP055164112, ISSN: 0140-7791, DOI: 10.1111/pce.12130
- M. ZHOU ET AL: "Constitutive Expression of a miR319 Gene Alters Plant Development and Enhances Salt and Drought Tolerance in Transgenic Creeping Bentgrass", PLANT PHYSIOLOGY, vol. 161, no. 3, 4 janvier 2013 (2013-01-04), pages 1375-1391, XP055164113, ISSN: 0032-0889, DOI: 10.1104/pp.112.208702
- BARDOU FLORIAN ET AL: "Dual RNAs in plants", BIOCHIMIE (PARIS),, vol. 93, no. 11, 1 novembre 2011 (2011-11-01), pages 1950-1954, XP002734738,
- CRAPPE J ET AL: "Little things make big things happen: A summary of micropeptide encoding genes", EUPA OPEN PROTEOMICS 2014 ELSEVIER NLD,, vol. 3, 1 janvier 2014 (2014-01-01), pages 128-137, XP002734739, ISSN: 2212-9685
- DE CONINCK BARBARA ET AL: "Mining the genome of Arabidopsis thaliana as a basis for the identification of novel bioactive peptides involved in oxidative stress tolerance", JOURNAL OF EXPERIMENTAL BOTANY,, vol. 64, no. 17, 1 décembre 2013 (2013-12-01), pages 5297-5307, XP002734740,
- DOMINIQUE LAURESSERGUES ET AL: "Primary transcripts of microRNAs encode regulatory peptides", NATURE, vol. 520, no. 7545, 25 mars 2015 (2015-03-25), pages 90-93, XP055225397, United Kingdom ISSN: 0028-0836, DOI: 10.1038/nature14346

### SEQUENCE LISTING

<110> Universit Toulouse III - Paul Sabatier
   Centre National de la Recherche Scientifique - CNRS
<120> UTILISATION DE MICROPEPTIDES POUR FAVORISER LA CROISSANCE DES PLANTES
<130> WOB 15 AY TTT M164
<150> FR 14 55045
   <151> 2014-06-03
<150> FR 14 55046
   <151> 2014-06-03
<150> FR 15 52469
   <151> 2015-03-24
<160> 12
<170> Patent In version 3.5
<210> 1
   <211> 21
   <212> RNA
   <213> Arabidopsis thaliana
<400> 1
   uggagaagca gggcacgugc a 21
<210> 2
   <211> 37
   <212> PRT
   <213> Arabidopsis thaliana
<400> 2
   Met Pro Ser Trp His Gly Met Val Leu Leu Pro Tyr Val Lys His Thr
   1               5                   10                  15
   His Ala Ser Thr His Thr His Thr His Asn Ile Tyr Gly Cys Ala Cys
               20                  25                  30
   Glu Leu Val Phe His
           35
<210> 3
   <211> 114
   <212> DNA
   <213> Arabidopsis thaliana
<400> 3
   atgccatcat ggcatggtat ggttcttttg ccttacgtaa aacacactca cgccagcaca       60
   cacacacaca cacataacat atacggatgt gcgtgtgagc tagtcttcca ttaa            114
<210> 4
   <211> 466
   <212> DNA
   <213> Arabidopsis thaliana
<400> 4
   agacaagccc ccacactaaa aaaacagtaa tatggaataa aaaaaagctt tcaaaactta 60
   gcagttatta gacaaggtat tgtttggccc tagctagcga tcgtttagct ctcttcactc 120
   tctcactttt ttagttcaac ccttcttttg cgtgagatgc catcatggca tggtatggtt 180
   cttttgcctt acgtaaaaca cactcacgcc agcacacaca cacacacaca taacatatac 240
   ggatgtgcgt gtgagctagt cttccattaa tgcaatcttt gggcctatat atacaaacct 300
   ttccataacc aaagttctca tactacaaac gcccctcatg tgcttggaaa tgcgggtgag 360
   aatctccatg ttggagaagc agggcacgtg caaaccaaca aacacgaaat ccgtctcatt 420
   tgcttatttg cacgtactta acttctccaa catgagctct tcaccc 466
<210> 5
   <211> 21
   <212> RNA
   <213> Arabidopsis thaliana
<400> 5
   ucggaccagg cuucaucccc c 21
<210> 6
   <211> 18
   <212> PRT
   <213> Arabidopsis thaliana
<400> 6
   Met Arg Val Lys Leu Phe Gln Leu Arg Gly Met Leu Ser Gly Ser Arg
   1 5 10 15
   Ile Leu
<210> 7
   <211> 57
   <212> DNA
   <213> Arabidopsis thaliana
<400> 7
   atgagggtta agctatttca gttgagggga atgttgtctg gatcgaggat attatag 57
<210> 8
   <211> 484
   <212> DNA
   <213> Arabidopsis thaliana
<400> 8
   ctagggttta ggaatgacga cctgtttctg ttgtgtctta ttaaaagccc atcttcgtct 60
<210> 9
   <211> 21
   <212> RNA
   <213> Arabidopsis thaliana
<400> 9
   uuggacugaa gggagcuccc u 21
<210> 10
   <211> 50
   <212> PRT
   <213> Arabidopsis thaliana
<400> 10
   Met Asn Ile His Thr Tyr His His Leu Leu Phe Pro Ser Leu Val Phe
   1               5                   10                  15
   His Gln Ser Ser Asp Val Pro Asn Ala Leu Ser Leu His Ile His Thr
               20                  25                  30
   Tyr Glu Tyr Ile Ile Val Val Ile Asp Pro Phe Arg Ile Thr Leu Ala
           35                  40                  45
   Phe Arg
       50
<210> 11
   <211> 153
   <212> DNA
   <213> Arabidopsis thaliana
<400> 11
   atgaatatac atacatacca tcatcttctt ttcccatctc tagtttttca tcaatcttct 60
   gatgttccaa acgctctatc tcttcatata catacatacg aatatattat tgttgtcata 120
   gatccattta gaatcacttt agcttttaga tga 153
<210> 12
   <211> 646
   <212> DNA
   <213> Arabidopsis thaliana
<400> 12
   ttgtatccgc agtgtatttc ctcgcatcta ccatcccttt tctacgcctc tctccctctc      60
   tctctttctc catcaaatct tgttttgttc aaactctctc tctctcatct attctctcca     120
   tacaatacat gaatatacat acataccatc atcttctttt cccatctcta gtttttcatc     180
   aatcttctga tgttccaaac gctctatctc ttcatataca tacatacgaa tatattattg     240
   ttgtcataga tccatttaga atcactttag cttttagatg agatctaggg tttctttgtt     300
   ttctttcaaa ttttgttgca tattcttcta aatcatggtt tttcgcttgc taggttatag     360
   atccatgcaa atatggagta gatgtacaaa cacacgctcg gacgcatatt acacatgttc     420
   atacacttaa tactcgctgt tttgaattga tgttttagga atatatatgt agagagagct     480
   tccttgagtc cattcacagg tcgtgatatg attcaattag cttccgactc attcatccaa     540
   ataccgagtc gccaaaattc aaactagact cgttaaatga atgaatgatg cggtagacaa     600
   attggatcat tgattctctt tgattggact gaagggagct ccctct                    646

## Description

La présente invention concerne l'utilisation de micropeptides (peptides codés par des microARNs ou « miPEPs ») pour favoriser la croissance des plantes.

Les microARNs (miRs) sont des petits ARNs non codants, d'environ 21 nucléotides après maturation, qui contrôlent l'expression de gènes cibles au niveau post-transcriptionnel, en dégradant l'ARNm cible ou en inhibant sa traduction.

Les miRs sont notamment rencontrés chez les plantes. Les gènes ciblés par les miRs sont souvent des gènes clés de processus développementaux. Par exemple, les miR164 ciblent des gènes de la famille CUC (Blein et al., Science, 322(5909): 1835-9, 2008). La famille des miR164 est ainsi impliquée dans le développement des méristèmes et notamment dans le développement de la feuille (Laufs et al., Development, 131(17):4311-22, 2004; Nikovics et al., Plant Cell, 18(11):2929-45, 2006). Dans un autre exemple, les miR319 ciblent des facteurs de transcription de la famille TCP, impliqués dans la croissance cellulaire (Nag et al., Proc Natl Acad Sci USA, 106(52):22534-9, 2009). La surexpression du miR319a conduit ainsi à un phénotype foliaire (phénotype *Jaw,* Palatnik et al., Nature, 425(6955) :257-63, 2003) de même qu'un phénotype floral (Nag *et al.,* 2009).

Les miRs pourraient donc, par leur action de régulation de l'expression de certains gènes, représenter une cible d'intérêt pour contrôler la croissance des plantes, et en particulier pour en favoriser la croissance.

La régulation de l'expression des miRs est très peu connue, mais il a été démontré que celle-ci fait intervenir, à l'instar de la plupart des gènes codants, une ARN polymerase II : cette enzyme produit un transcrit primaire, appelé «*pri-miR*», qui est ensuite maturé par un complexe protéique contenant notamment les enzymes type Dicer. Cette maturation conduit tout d'abord à la formation d'un précurseur de miR appelé «*pre-miR*», ayant une structure secondaire en forme tige-boucle contenant le *miR* et sa séquence *miR** complémentaire. Puis le précurseur est maturé, ce qui conduit à la formation d'un ARN double brin plus court contenant le miR et le miR*. Le miR est ensuite pris en charge par le complexe RISC qui clive l'ARNm du gène cible ou bien inhibe sa traduction.

Jusqu'à présent, les miRs, et par extension leur transcrit primaire, ont toujours été considérés, de par leur mode d'action particulier, comme des ARNs régulateurs non codants et ne produisant aucun peptide. Or, les Inventeurs ont récemment mis en évidence dans la demande de brevet FR 13/60727 l'existence de micropeptides (ou « miPEPs », *microRNA encoded PEPtides)* capables de moduler l'accumulation des miRs.

Dans ce contexte, la présente invention a pour but de proposer de nouveaux outils efficaces et écologiques pour favoriser la croissance des plantes.

L'un des aspects de l'invention est de proposer une nouvelle utilisation des miPEPs pour favoriser la croissance des plantes.
Un autre aspect de l'invention concerne également un nouveau procédé de culture de plantes.
Un autre aspect de l'invention est de proposer une composition de miPEPs permettant de favoriser la croissance des plantes.
L'un des autres aspects de l'invention est également de proposer une plante transgénique et des parties de plantes transgéniques, et leur procédé de production.
L'un des autres aspects de l'invention est également de proposer des organes, cellules et semences de plantes transgéniques.
L'un des autres aspects de l'invention est de proposer des plantes modifiées écologiquement.

L'invention concerne donc l'utilisation d'un peptide pour favoriser la croissance d'une plante, ledit peptide étant introduit dans la plante, ledit peptide ayant une séquence d'acides aminés comprenant ou consistant en une séquence identique à celle d'un miPEP naturellement présent dans ladite plante,
ledit miPEP naturellement présent dans ladite plante étant un peptide de 3 à 100 acides aminés, notamment 4 à 100 acides aminés, dont la séquence est codée par un cadre ouvert de lecture situé sur le transcrit primaire d'un miR, ledit miPEP étant capable de moduler l'accumulation dudit miR dans ladite plante, lequel miR régule l'expression d'au moins un gène impliqué dans le développement des parties végétatives ou reproductrices de la plante, notamment les racines, la tige, les feuilles ou les fleurs.

De manière surprenante et inattendue, les Inventeurs ont constaté que l'utilisation de peptides dont la séquence comprend ou consiste en une séquence identique à celle de miPEPs codés sur les transcrits primaires de miRs, permet de favoriser la croissance des plantes.

Dans l'invention, les termes « *microARN », « microARN non codant »* et « *miR* » sont équivalents et peuvent être utilisés l'un pour l'autre. Ils définissent des petites molécules d'ARN d'environ 21 nucléotides, qui ne sont pas traduites et ne conduisent pas à un peptide ou une protéine. Cependant, sous cette forme mature, les miRs assurent une fonction de régulation de certains gènes *via* des mécanismes post-transcriptionnels, comme par exemple par l'intermédiaire du complexe RISC.

Le « *transcrit primaire de miR* » (ou « *pri-miR* ») correspond lui à la molécule d'ARN directement obtenue à partir de la transcription de la molécule d'ADN. Généralement, ce transcrit primaire subit une ou plusieurs modifications post-transcriptionnelles, qui entraînent par exemple une structure particulière de l'ARN ou un clivage de certaines parties de l'ARN par des phénomènes d'épissage, et qui conduisent à la forme précurseur du miR ou (*« pre-miR* »), puis à la forme mature du miR.

Les termes « *micropeptides »* et « *miPEPs »* (*microRNA encoded PEPtides*) sont équivalents et peuvent être utilisés l'un pour l'autre. Ils définissent un peptide qui est codé par un cadre ouvert de lecture présent sur le transcrit primaire d'un miR, et qui est capable de moduler l'accumulation dudit miR. Les miPEPs au sens de la présente invention ne doivent pas être entendus comme étant nécessairement des peptides de petite taille, dans la mesure où « micro » ne correspond pas à la taille du peptide.

Comme indiqué dans la demande de brevet FR 13/60727, les miPEPs sont des peptides :
- de 4 à 100 acides aminés, de préférence de 4 à 60 acides aminés, notamment de 4 à 59 acides aminés,
- codés par un cadre ouvert de lecture contenu dans le transcrit primaire d'un miR, de préférence dans la partie 5' du transcrit primaire dudit miR, et
- capables de moduler l'accumulation dudit miR dans une cellule eucaryote.

Les termes « *cadre ouvert de lecture »* ou « *ORF »* (*open reading frame*) sont équivalents et peuvent être utilisés l'un pour l'autre. Ils correspondent à une séquence de nucléotides dans une molécule d'ADN ou d'ARN pouvant potentiellement coder un peptide ou une protéine: ledit cadre ouvert de lecture débute par un codon start (le codon start codant généralement une méthionine), suivi d'une série de codons (chaque codon codant un acide aminé), et se termine par un codon stop (le codon stop n'étant pas traduit).
Dans l'invention, les ORFs peuvent être dénommés de manière spécifique *« miORFs »* lorsque ceux-ci sont présents sur les transcrits primaires de miR.

Les miORFs tels que définis dans l'invention peuvent avoir une taille de 15 à 303 nucléotides. Un acide aminé étant codé par un codon de 3 nucléotides, les miORFs de 15 à 303 nucléotides codent des miPEPS de 4 à 100 acides aminés.
En particulier, les miORFs ont une taille de :
15, 18, 21, 24, 27, 30, 33, 36, 39, 42, 45, 47, 51, 54, 57, 60, 63, 66, 69, 72, 75, 78, 81, 84, 87, 90, 93, 96, 99, 102, 105, 108, 111, 114, 117, 120, 123, 126, 129, 132, 135, 138, 141, 144, 147, 150, 153, 156, 159, 162, 165, 168, 171, 174, 177, 180, 183, 186, 189, 192, 195, 198, 201, 204, 207, 210, 213, 216, 219, 222, 225, 228, 231, 234, 237, 240, 243, 246, 249, 252, 255, 258, 261, 264, 267, 270, 273, 276, 279, 282, 285, 288, 291, 294, 297, 300 ou 303 nucléotides, et codent respectivement des miPEPs ayant une taille de :
4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99 ou 100 acides aminés.

Un miPEP peut également avoir une taille de 3 acides aminés.

Compte tenu de la dégénérescence du code génétique, un même miPEP peut être codé par plusieurs séquences nucléotidiques. De telles séquences nucléotidiques, différentes entre elles d'au moins un nucléotide mais codant un même peptide, sont appelées « *séquences dégénérées ».*

Dans l'invention, le terme « *plante* » fait référence de manière générale à tout ou partie d'une plante quel que soit son stade de développement (y compris la plante sous forme de graine ou de jeune pousse), à un ou plusieurs organes de la plante (comme par exemple les feuilles, les racines, la tige, les fleurs), à une ou plusieurs cellules de la plante, ou encore à un amas de cellules de la plante.
Dans l'invention, le terme *« croissance »* fait référence au développement de tout ou partie d'une plante au cours du temps. La croissance de la plante peut ainsi être déterminée et quantifiée par le suivi de paramètres évolutifs observables pour certaines parties, cellules ou organes de la plante, tels que les feuilles, les racines, les tiges ou encore les fleurs.

De manière non limitative, les paramètres permettant de déterminer et quantifier la croissance d'une plante peuvent être notamment :
- la taille, la surface, le volume, la masse et le nombre de feuilles,
- la forme des feuilles,
- la taille et le nombre de fleurs,
- la taille de la tige (ou de la hampe florale),
- la biomasse racinaire
- le nombre, la longueur et le niveau de ramification des racines,
- la précocité de la germination,
- la vigueur germinative et la durée de phase juvénile,
- la précocité du bourgeonnement,
- la précocité de l'induction florale (ou transition florale),
- ou encore le nombre de cellules.

Par ailleurs, dans l'invention, l'expression *« favoriser la croissance de la plante* », ou *« améliorer la croissance de la plante* », indique :
- soit une accélération du développement (comme par exemple une taille des feuilles plus importante pour une plante à un instant donné par rapport à une plante de référence),
- soit à un accroissement du développement (comme par exemple une taille de feuilles plus importante pour une plante qui ne peut être atteinte par une plante de référence),
- soit à une accélération et un accroissement du développement de la plante.

Il est important de noter que l'utilisation selon l'invention possède l'avantage d'être écologique, en comparaison des méthodes chimiques classiquement utilisées dans l'industrie botanique ou dans l'agriculture, car le miPEP est un peptide qui est présent naturellement chez la plante.

L'invention concerne également l'utilisation d'un miPEP introduit par voie exogène dans une plante pour en favoriser la croissance,
ledit miPEP introduit par voie exogène étant un peptide comprenant, ou consistant en, une séquence identique à celle d'un miPEP naturellement présent dans ladite plante,
lequel miPEP naturellement présent est un peptide de 3 à 100 acides aminés, notamment de 4 à 100 acides aminés, dont la séquence est codée par un cadre ouvert de lecture situé en 5' sur le transcrit primaire d'un miR,
ledit miPEP étant capable de moduler l'accumulation dudit miR dans ladite plante, lequel miR régule l'expression d'au moins un gène impliqué dans le développement des parties végétatives ou reproductrices de la plante, notamment les racines, la tige, les feuilles ou les fleurs,
la somme de la quantité dudit miPEP introduit par voie exogène et de celle dudit miPEP naturellement présent étant strictement supérieure à la quantité dudit miPEP naturellement présent.

Dans l'invention, l'expression « *miPEP introduit par voie exogène* » fait référence à un miPEP introduit artificiellement dans la plante, que celui-ci existe ou non naturellement dans la plante.

Si le miPEP existe naturellement chez la plante, il s'agit d'un *« miPEP d'origine endogène ».*

Si le miPEP n'existe pas naturellement chez la plante, il s'agit d'un « *miPEP d'origine exogène ».*
Lorsqu'il est introduit dans la plante un « *miPEP d'origine exogène* », il est alors nécessaire d'introduire également le miR correspondant et son transcrit primaire.

L'introduction d'un miPEP par voie exogène dans la plante implique donc une étape technique, laquelle étape n'est pas un phénomène naturel et ne correspond ni à un croisement, ni à une sélection.

Le miPEP introduit par voie exogène peut être soit un peptide produit hors de la plante (comme par exemple un peptide isolé et/ou purifié, un peptide synthétique ou un peptide recombinant), soit un peptide produit dans la plante suite à l'introduction non naturelle d'un acide nucléique codant ledit miPEP dans ladite plante.

La plante dans laquelle le miPEP n'a pas été introduit possède une quantité basale dudit miPEP, qui correspond à celle dudit miPEP naturellement présent. L'utilisation d'un miPEP comprenant, ou consistant en, une séquence identique à celle dudit miPEP entraîne une augmentation de la quantité totale de miPEP, ce qui module l'accumulation du miR dont le transcrit primaire contient la séquence codant ledit miPEP.

Par ailleurs, le miPEP introduit se retrouve dans la plante et son introduction n'a pas d'impact sur sa stabilité.

Dans l'invention, par « *accumulation »,* on entend la production d'une molécule, telle qu'un miR ou un miPEP, dans la cellule.
Ainsi, la « *modulation de l'accumulation* » d'une molécule dans une cellule correspond à une modification de la quantité de cette molécule dans la cellule.

Dans un mode de réalisation, l'invention concerne l'utilisation telle que définie ci-dessus, dans lequel la modulation de l'accumulation dudit miR est une diminution ou une augmentation de l'accumulation dudit miR, en particulier une augmentation.

Une « *diminution de l'accumulation du miR »* correspond à une baisse de la quantité de ladite molécule dans la cellule.
A l'inverse, une *« augmentation de l'accumulation du miR »* correspond à une hausse de la quantité de ladite molécule dans la cellule.

Dans un mode de réalisation, l'invention concerne l'utilisation telle que définie ci-dessus, dans laquelle ledit gène, impliqué dans le développement des parties végétatives ou reproductrices de la plante, est choisi parmi le groupe consistant en : *NAC1* (Accession n°AT1G56010.1), *NAC4* (Accession n° AT5G07680.1), *NAC5* (Accession n° AT5G61430.1), *CUC1* (Accession n° AT3G15170.1) et *CUC2* (Accession n° AT5G53950.1) (numéros d'accession selon la banque de données *The Arabidopsis Information Resource « TAIR »).*

Dans un mode de réalisation, l'invention concerne l'utilisation telle que définie ci-dessus, dans laquelle ledit miARN est le miR164a, en particulier, dans laquelle ledit miR164a possède une séquence nucléotidique consistant en SEQ ID NO : 1.

Dans un mode de réalisation, l'invention concerne l'utilisation telle que définie ci-dessus, dans laquelle ledit miARN est le miR164a, en particulier, dans laquelle ledit miR164a possède une séquence nucléotidique consistant en SEQ ID NO : 1, et ledit gène, impliqué dans le développement des parties végétatives ou reproductrices de la plante, est choisi parmi le groupe consistant en : *NAC1* (Accession n°AT1G56010.1), *NAC4* (Accession n° AT5G07680.1), *NAC5* (Accession n° AT5G61430.1), *CUC1* (Accession n° AT3G15170.1) et *CUC2* (Accession n° AT5G53950.1) (numéros d'accession selon la banque de données *The Arabidopsis Information Resource « TAIR »).*

En particulier, l'invention concerne l'utilisation telle que définie ci-dessus, dans laquelle ledit ledit miR164a possède une séquence nucléotidique présentant au moins 80% d'identité, de préférence au moins 90 % d'identité, avec la séquence nucléotidique SEQ ID NO : 1.

Dans un mode de réalisation, l'invention concerne l'utilisation telle que définie ci-dessus, dans laquelle ledit miPEP est le miPEP164a, en particulier, dans laquelle ledit miPEP164a possède une séquence d'acides aminés consistant en la séquence SEQ ID NO : 2.

En particulier, l'invention concerne l'utilisation telle que définie ci-dessus, dans laquelle ledit ledit miPEP164a possède une séquence d'acides aminés présentant au moins 80% d'identité, de préférence au moins 90 % d'identité, avec la séquence d'acides aminés SEQ ID NO : 2.

Dans un mode de réalisation, l'invention concerne l'utilisation telle que définie ci-dessus, dans laquelle ledit gène, impliqué dans le développement des parties végétatives ou reproductrices de la plante, est choisi parmi le groupe consistant en: *REVOLUTA* (Accession n° AT5G60690), *PHABULOSA* (Accession n° AT2G34710), *PHAVOLUTA* (Accession n° AT1G30490), *ATHB-8* (Accession n° AT4G32880) et *ATHB-15* (Accession n° AT1G52150) (numéros d'accession selon la banque de données *The Arabidopsis Information Resource « TAIR »).*

Dans un mode de réalisation, l'invention concerne l'utilisation telle que définie ci-dessus, dans laquelle ledit miARN est le miR165a, en particulier, dans laquelle ledit miR165a possède une séquence nucléotidique consistant en SEQ ID NO : 5.

Dans un mode de réalisation, l'invention concerne l'utilisation telle que définie ci-dessus, dans laquelle ledit miARN est le miR165a, en particulier, dans laquelle ledit miR165a possède une séquence nucléotidique consistant en SEQ ID NO : 5, et ledit gène, impliqué dans le développement des parties végétatives ou reproductrices de la plante, est choisi parmi le groupe consistant en: *REVOLUTA* (Accession n° AT5G60690), *PHABULOSA* (Accession n° AT2G34710), *PHAVOLUTA* (Accession n° AT1G30490), *ATHB-8* (Accession n° AT4G32880) et *ATHB-15* (Accession n° AT1G52150) (numéros d'accession selon la banque de données *The Arabidopsis Information Resource « TAIR »*).

En particulier, l'invention concerne l'utilisation telle que définie ci-dessus, dans laquelle ledit ledit miR165a possède une séquence nucléotidique présentant au moins 80% d'identité, de préférence au moins 90 % d'identité, avec la séquence nucléotidique SEQ ID NO: 5.

Dans un mode de réalisation, l'invention concerne l'utilisation telle que définie ci-dessus, dans laquelle ledit miPEP est le miPEP165a, en particulier, dans laquelle ledit miPEP165a possède une séquence d'acides aminés consistant en la séquence SEQ ID NO : 6.

En particulier, l'invention concerne l'utilisation telle que définie ci-dessus, dans laquelle ledit ledit miPEP165a possède une séquence d'acides aminés présentant au moins 80% d'identité, de préférence au moins 90 % d'identité, avec la séquence d'acides aminés SEQ ID NO : 6.

Dans un mode de réalisation, l'invention concerne l'utilisation telle que définie ci-dessus, dans laquelle ledit gène, impliqué dans le développement des parties végétatives ou reproductrices de la plante, est choisi parmi le groupe consistant en: *TCP3* (Accession n°AT1G53230.1) et *TCP4* (Accession n°AT3G15030.1) (numéros d'accession selon la banque de données *The Arabidopsis Information Resource « TAIR »).*

Dans un mode de réalisation, l'invention concerne l'utilisation telle que définie ci-dessus, dans laquelle ledit miARN est le miR319a, en particulier, dans laquelle ledit miR319a possède une séquence nucléotidique consistant en la séquence SEQ ID NO : 9.

Dans un mode de réalisation, l'invention concerne l'utilisation telle que définie ci-dessus, dans laquelle ledit miARN est le miR319a, en particulier, dans laquelle ledit miR319a possède une séquence nucléotidique consistant en SEQ ID NO : 9, et ledit gène, impliqué dans le développement des parties végétatives ou reproductrices de la plante, est choisi parmi le groupe consistant en: *TCP3* (Accession n°AT1G53230.1) et *TCP4* (Accession n°AT3G15030.1 (numéros d'accession selon la banque de données *The Arabidopsis Information Resource « TAIR »).*

En particulier, l'invention concerne l'utilisation telle que définie ci-dessus, dans laquelle ledit miR319a possède une séquence nucléotidique présentant au moins 80% d'identité, de préférence au moins 90 % d'identité, avec la séquence nucléotidique SEQ ID NO : 9.

Dans un mode de réalisation, l'invention concerne l'utilisation telle que définie ci-dessus, dans laquelle ledit miPEP est le miPEP319a, en particulier, dans laquelle ledit miPEP319a possède une séquence d'acides aminés consistant en la séquence SEQ ID NO : 10.

En particulier, l'invention concerne l'utilisation telle que définie ci-dessus, dans laquelle ledit miPEP319a possède une séquence d'acides aminés présentant au moins 80% d'identité, de préférence au moins 90 % d'identité, avec la séquence d'acides aminés SEQ ID NO : 10.

Dans un mode de réalisation, l'invention concerne l'utilisation telle que définie ci-dessus, dans laquelle ladite plante est une plante crucifère telle *qu'Arabidopsis thaliana,* une plante légumineuse telle que *Glycine max* (soja), *Medicago truncatula* et *Medicago sativa* (luzerne) ou une plante solanacée telle que *Nicotiana benthamiana* (tabac), *Solanum tuberosum* (pomme de terre), *Solanum lycopersicum* (tomate) ou *Solanum melongena* (aubergine).

Dans un mode de réalisation, l'invention concerne l'utilisation telle que définie ci-dessus, dans laquelle ladite plante est une plante crucifère.

Dans un mode de réalisation, l'invention concerne l'utilisation telle que définie ci-dessus, dans laquelle ladite plante est une plante crucifère et le miR est le miR164a.

Dans un mode de réalisation, l'invention concerne l'utilisation telle que définie ci-dessus, dans laquelle ladite plante est une plante crucifère et le miR est le miR165a.

Dans un mode de réalisation, l'invention concerne l'utilisation telle que définie ci-dessus, dans laquelle ladite plante est une plante crucifère et le miR est le miR319a.

Dans un mode de réalisation, l'invention concerne l'utilisation telle que définie ci-dessus, dans laquelle ladite plante est *Arabidopsis thaliana.*

Dans un mode de réalisation, l'invention concerne l'utilisation telle que définie ci-dessus, pour favoriser la croissance d'une plante *Arabidopsis thaliana,* dans laquelle le miPEP164a est introduit par voie exogène dans ladite plante *Arabidopsis thaliana,* ledit miPEP164a étant également naturellement présent dans ladite plante *Arabidopsis thaliana,*
ledit miPEP164a introduit par voie exogène étant un peptide dont la séquence comprend ou consiste en une séquence identique à celle dudit miPEP164a naturellement présent, ladite séquence du miPEP164a naturellement présent étant codée par un cadre ouvert de lecture situé en 5' sur le transcrit primaire du miR164a, lequel miR164a régule l'expression d'au moins un gène impliqué dans le développement des parties végétatives ou reproductrices d'*Arabidopsis thaliana,*
la somme de la quantité dudit miPEP164a introduit par voie exogène et de celle dudit miPEP164a naturellement présent étant strictement supérieure à la quantité dudit miPEP164a naturellement présent chez ladite plante *Arabidopsis thaliana.*

Dans un mode de réalisation, l'invention concerne l'utilisation telle que définie ci-dessus, pour favoriser la croissance d'une plante *Arabidopsis thaliana,* dans laquelle le miPEP165a est introduit par voie exogène dans ladite plante *Arabidopsis thaliana,* ledit miPEP165a étant également naturellement présent dans ladite plante *Arabidopsis thaliana,*
ledit miPEP165a introduit par voie exogène étant un peptide dont la séquence comprend ou consiste en une séquence identique à celle dudit miPEP165a naturellement présent, ladite séquence du miPEP165a naturellement présent étant codée par un cadre ouvert de lecture situé en 5' sur le transcrit primaire du miR165a, lequel miR165a régule l'expression d'au moins un gène impliqué dans le développement des parties végétatives ou reproductrices *d'Arabidopsis thaliana,*
la somme de la quantité dudit miPEP165a introduit par voie exogène et de celle dudit miPEP165a naturellement présent étant strictement supérieure à la quantité dudit miPEP165a naturellement présent chez ladite plante *Arabidopsis thaliana.*

Dans un mode de réalisation, l'invention concerne l'utilisation telle que définie ci-dessus, pour favoriser la croissance d'une plante *Arabidopsis thaliana,* dans laquelle le miPEP319a est introduit par voie exogène dans ladite plante *Arabidopsis thaliana,* ledit miPEP319a étant également naturellement présent dans ladite plante *Arabidopsis thaliana,*
ledit miPEP319a introduit par voie exogène étant un peptide dont la séquence comprend ou consiste en une séquence identique à celle dudit miPEP319a naturellement présent, ladite séquence du miPEP319a naturellement présent étant codée par un cadre ouvert de lecture situé en 5' sur le transcrit primaire du miR319a, lequel miR319a régule l'expression d'au moins un gène impliqué dans le développement des parties végétatives ou reproductrices d'*Arabidopsis thaliana,*
la somme de la quantité dudit miPEP319a introduit par voie exogène et de celle dudit miPEP319a naturellement présent étant strictement supérieure à la quantité dudit miPEP319a naturellement présent chez ladite plante *Arabidopsis thaliana.*

Dans un mode de réalisation, l'invention concerne l'utilisation telle que définie ci-dessus, dans laquelle ledit miPEP est introduit par voie externe dans la plante, de préférence par arrosage, par pulvérisation ou par l'ajout d'un engrais, d'un terreau, d'un substrat de culture ou d'un support en contact avec la plante.

Dans un mode de réalisation, l'invention concerne l'utilisation telle que définie ci-dessus, dans laquelle ledit miPEP est introduit par voie externe dans une graine ou une semence, de préférence par arrosage, par pulvérisation ou par l'ajout d'un engrais, d'un terreau, d'un substrat de culture ou d'un support en contact avec la graine ou la semence.

Dans un mode de réalisation, l'invention concerne le procédé tel que défini ci-dessus, dans lequel ledit miPEP est utilisé pour traiter la plante sous forme de graine ou de semence.

Dans un mode de réalisation, l'invention concerne l'utilisation telle que définie ci-dessus, dans laquelle ledit miPEP est introduit par arrosage et par pulvérisation.

Dans un mode de réalisation, l'invention concerne l'utilisation telle que définie ci-dessus, dans laquelle ledit miPEP est introduit par arrosage et par l'ajout d'un engrais.

Dans un mode de réalisation, l'invention concerne l'utilisation telle que définie ci-dessus, dans laquelle ledit miPEP est introduit par pulvérisation et par l'ajout d'un engrais.

Dans un mode de réalisation, l'invention concerne l'utilisation telle que définie ci-dessus, dans laquelle ledit miPEP est introduit, par arrosage, par pulvérisation et par l'ajout d'un engrais.

Les Inventeurs ont en effet constaté de manière inattendue qu'il est possible d'appliquer directement une composition comprenant un miPEP sur la plante pour moduler l'accumulation du miR correspondant dans la plante, ce qui indique que le miPEP est capté par la plante.

Dans un mode de réalisation, l'invention concerne l'utilisation telle que définie ci-dessus, dans laquelle la plante est traitée avec une composition comprenant 10⁻⁹ M à 10⁻⁴ M dudit miPEP, notamment 10⁻⁹ M, 10⁻⁸ M, 10⁻⁷ M, 10⁻⁶ M, 10⁻⁵ M ou 10⁻⁴ M dudit miPEP.

De préférence, les compositions ont une concentration de 10⁻⁸ M à 10⁻⁵ M pour une application par arrosage ou par pulvérisation sur la plante.

De manière complémentaire, des compositions plus ou moins concentrées peuvent être envisagées pour traiter la plante avec le miPEP. Par exemple, et de manière non limitative, des compositions plus concentrées comprenant 10⁻¹ M à 10⁻³ M, notamment 10⁻² M de miPEP, peuvent être utilisées dans le cas où le miPEP introduit par voie exogène est administré à la plante par épandage.

Les propriétés de solubilité des miPEPs sont déterminées notamment par leur composition en acides aminés. Les miPEPs hydrophiles peuvent être solubilisés et conditionnés dans des solutions aqueuses, telles que l'eau. Les miPEPs hydrophobes peuvent être solubilisés et conditionnés dans des solvants, tels que les solvants organiques.

Pour un traitement des plantes par les miPEPs, les solvants organiques sont des solvants non toxiques pour les plantes en faibles quantités, c'est-à-dire qu'ils n'ont pas d'effet délétères sur le développement de la plante. De manière non limitative, les solvants organiques peuvent être choisis parmi l'acétonitrile et l'acide acétique.

Les miPEPs peuvent également être solubilisés et conditionnés dans des mélanges de solvants organiques, comme par exemple un mélange d'acétonitrile et d'acide acétique. En particulier, les miPEPs peuvent être solubilisés dans une solution comprenant 50 % d'acétonitrile, 10% d'acide acétique et 40% d'eau (volume/volume/volume).

En particulier, le miPEP164a est solubilisé dans l'eau.
En particulier, le miPEP164a est à une concentration de 10⁻⁹ M à 10⁻⁴ M dans de l'eau.

En particulier, le miPEP165a est solubilisé dans *l'eau.*
En particulier, le miPEP165a est à une concentration de 10⁻⁹ M à 10⁻⁴ M dans de l'eau.

En particulier, le miPEP319a est solubilisé dans une solution comprenant 50% d'acétonitrile, 10 % d'acide acétique et 40 % d'eau (volume/volume/volume).

En particulier, le miPEP319a solubilisé dans une solution comprenant 50% d'acétonitrile, 10 % d'acide acétique et 40 % d'eau (volume/volume/volume) est dilué à une concentration de 10⁻⁹ M à 10⁻⁴ M avec de l'eau.

Dans un mode de réalisation, l'invention concerne l'utilisation telle que définie ci-dessus, dans laquelle ledit miPEP est introduit dans la plante par le biais d'un acide nucléique codant ledit miPEP, ledit acide nucléique étant introduit dans la plante.

Dans un mode de réalisation, l'invention concerne l'utilisation telle que définie ci-dessus, dans laquelle la taille de la tige est augmentée chez la plante dans laquelle a été introduit ledit miPEP par rapport à la taille de la tige d'une plante identique et de même âge dans laquelle aucun miPEP n'a été introduit, ou bien par rapport à la taille de la tige d'une plante identique et de même âge dans laquelle ledit miPEP n'a pas été introduit.

Dans un mode de réalisation, l'invention concerne l'utilisation telle que définie ci-dessus, dans laquelle le nombre de feuilles est augmenté chez la plante dans laquelle a été introduit ledit miPEP par rapport au nombre de feuilles d'une plante identique et de même âge dans laquelle aucun miPEP n'a été introduit, ou bien par rapport au nombre de feuilles d'une plante identique et de même âge dans laquelle ledit miPEP n'a pas été introduit.

Dans un mode de réalisation, l'invention concerne l'utilisation telle que définie ci-dessus, dans laquelle la taille des feuilles est augmentée chez la plante dans laquelle a été introduit ledit miPEP par rapport à la taille des feuilles d'une plante identique et de même âge dans laquelle aucun miPEP n'a été introduit, ou bien par rapport à la taille des feuilles d'une plante identique et de même âge dans laquelle ledit miPEP n'a pas été introduit.

Dans un mode de réalisation, l'invention concerne l'utilisation telle que définie ci-dessus, dans laquelle le nombre de racines est augmenté chez la plante dans laquelle a été introduit ledit miPEP par rapport au nombre de racines d'une plante identique et de même âge dans laquelle aucun miPEP n'a été introduit, ou bien par rapport au nombre de racines d'une plante identique et de même âge dans laquelle ledit miPEP n'a pas été introduit.

Dans un mode de réalisation, l'invention concerne l'utilisation telle que définie ci-dessus, dans laquelle la longueur des racines est augmentée chez la plante dans laquelle a été introduit ledit miPEP par rapport à la longueur des racines d'une plante identique et de même âge dans laquelle aucun miPEP n'a été introduit, ou bien par rapport à la longueur des racines d'une plante identique et de même âge dans laquelle ledit miPEP n'a pas été introduit.

Dans un mode de réalisation, l'invention concerne l'utilisation telle que définie ci-dessus, dans laquelle le nombre de fleurs est augmenté chez la plante dans laquelle a été introduit ledit miPEP par rapport au nombre de fleurs d'une plante identique et de même âge dans laquelle aucun miPEP n'a été introduit, ou bien par rapport au nombre de fleurs d'une plante identique et de même âge dans laquelle ledit miPEP n'a pas été introduit.

Dans un mode de réalisation, l'invention concerne l'utilisation telle que définie ci-dessus, dans laquelle la date de floraison est avancée chez la plante dans laquelle a été introduit ledit miPEP par rapport à la date de floraison d'une plante identique et de même âge dans laquelle aucun miPEP n'a été introduit, ou bien par rapport à la date de floraison d'une plante identique et de même âge dans laquelle ledit miPEP n'a pas été introduit.

Dans un mode de réalisation, l'invention concerne l'utilisation telle que définie ci-dessus, dans laquelle la taille de la hampe florale est augmentée chez la plante dans laquelle a été introduit ledit miPEP par rapport à la taille de la hampe florale d'une plante identique et de même âge dans laquelle aucun miPEP n'a été introduit, ou bien par rapport à la taille de la hampe florale d'une plante identique et de même âge dans laquelle ledit miPEP n'a pas été introduit.

L'augmentation des paramètres permettant de déterminer et quantifier la croissance chez la plante dans laquelle a été introduit le miPEP (tels que la taille de la tige, le nombre et la taille des feuilles, ou encore le nombre et la longueur des racines) est de préférence mis en évidence par comparaison avec une plante identique (c'est-à-dire une plante de la même espèce et/ou variété), de même âge et cultivée dans les mêmes conditions mais dans laquelle aucun miPEP n'a été introduit.

L'invention concerne également l'utilisation d'un miPEP introduit par voie exogène dans une plante pour en favoriser la croissance,
ledit miPEP étant codé par le transcrit primaire, introduit artificiellement dans la plante, d'un miR,
ledit transcrit primaire, ledit miR et ledit miPEP étant absents naturellement chez la plante, ledit miPEP étant capable de moduler l'accumulation dudit miR dans ladite plante, lequel miR régule l'expression d'au moins un gène impliqué dans le développement des parties végétatives ou reproductrices de la plante, notamment les racines, la tige, les feuilles ou les fleurs.

Dans un mode de réalisation particulier, ledit transcrit primaire du miR, le miR et ledit miPEP sont introduits dans la plante à l'aide d'un vecteur.

Dans un autre aspect, l'invention concerne un procédé pour favoriser la croissance d'une plante, comprenant une étape d'introduction d'un miPEP dans une plante par voie exogène, ledit miPEP étant également présent naturellement dans ladite plante,
ledit miPEP introduit par voie exogène étant un peptide de 3 à 100 acides aminés, notamment de 4 à 100 acides aminés dont la séquence comprend ou consiste en une séquence identique à celle dudit miPEP naturellement présent, laquelle séquence du miPEP naturellement présent est codée par un cadre ouvert de lecture situé en 5' sur le transcrit primaire d'un miR, ledit miPEP étant capable de moduler l'accumulation dudit miR, lequel miR régule l'expression d'au moins un gène impliqué dans le développement des parties végétatives ou reproductrices de la plante, notamment les racines, la tige, les feuilles ou les fleurs,
la somme de la quantité dudit miPEP introduit par voie exogène et de celle dudit miPEP naturellement présent étant strictement supérieure à la quantité dudit miPEP naturellement présent.

Dans un mode de réalisation, l'invention concerne un procédé tel que défini ci-dessus, dans lequel ledit gène, impliqué dans le développement des parties végétatives ou reproductrices de la plante, est choisi parmi le groupe consistant en : *NAC1* (Accession n°AT1G56010.1), *NAC4* (Accession n° AT5G07680.1), *NAC5* (Accession n° AT5G61430.1), *CUC1* (Accession n° AT3G15170.1) et *CUC2* (Accession n° AT5G53950.1).

Dans un mode de réalisation, l'invention concerne un procédé tel que défini ci-dessus, dans lequel ledit miARN est le miR164a, en particulier, dans lequel ledit miR164a possède une séquence nucléotidique consistant en SEQ ID NO : 1.

Dans un mode de réalisation, l'invention concerne un procédé tel que défini ci-dessus, dans lequel ledit miARN est le miR164a, en particulier, dans lequel ledit miR164a possède une séquence nucléotidique consistant en SEQ ID NO : 1, et ledit gène, impliqué dans le développement des parties végétatives ou reproductrices de la plante, est choisi parmi le groupe consistant en: *NAC1* (Accession n°AT1G56010.1), *NAC4* (Accession n° AT5G07680.1), *NAC5* (Accession n° AT5G61430.1), *CUC1* (Accession n° AT3G15170.1) et *CUC2* (Accession n° AT5G53950.1).

Dans un mode de réalisation, l'invention concerne un procédé tel que défini ci-dessus, dans lequel ledit miPEP est le miPEP164a, en particulier, dans lequel ledit miPEP164a possède une séquence d'acides aminés consistant en SEQ ID NO : 2.

Dans un mode de réalisation, l'invention concerne un procédé tel que défini ci-dessus, dans lequel ledit gène, impliqué dans le développement des parties végétatives ou reproductrices de la plante, est choisi parmi le groupe consistant en: *REVOLUTA* (Accession n° AT5G60690), *PHABULOSA* (Accession n° AT2G34710), *PHAVOLUTA* (Accession n° AT1G30490), *ATHB-8* (Accession n° AT4G32880) et *ATHB-15* (Accession n° AT1G52150).

Dans un mode de réalisation, l'invention concerne un procédé tel que défini ci-dessus, dans lequel ledit miARN est le miR165a, en particulier, dans lequel ledit miR165a possède une séquence nucléotidique consistant en SEQ ID NO : 5.

Dans un mode de réalisation, l'invention concerne un procédé tel que défini ci-dessus, dans lequel ledit miARN est le miR165a, en particulier, dans lequel ledit miR165a possède une séquence nucléotidique consistant en SEQ ID NO : 5, et ledit gène, impliqué dans le développement des parties végétatives ou reproductrices de la plante, est choisi parmi le groupe consistant en: *REVOLUTA* (Accession n° AT5G60690), *PHABULOSA* (Accession n° AT2G34710), *PHAVOLUTA* (Accession n° AT1G30490), *ATHB-8* (Accession n° AT4G32880) et *ATHB-15* (Accession n° AT1G52150).

Dans un mode de réalisation, l'invention concerne un procédé tel que défini ci-dessus, dans lequel ledit miPEP est le miPEP165a, en particulier, dans lequel ledit miPEP165a possède une séquence d'acides aminés consistant en SEQ ID NO : 6.

Dans un mode de réalisation, l'invention concerne un procédé tel que défini ci-dessus, dans lequel ledit gène, impliqué dans le développement des parties végétatives ou reproductrices de la plante, est choisi parmi le groupe consistant en : *TCP3* (Accession n°AT1G53230.1) et *TCP4* (Accession n°AT3G15030.1).

Dans un mode de réalisation, l'invention concerne un procédé tel que défini ci-dessus, dans lequel ledit miARN est le miR319a, en particulier, dans lequel ledit miR319a possède une séquence nucléotidique consistant en SEQ ID NO : 9.

Dans un mode de réalisation, l'invention concerne un procédé tel que défini ci-dessus, dans lequel ledit miARN est le miR319a, en particulier, dans lequel ledit miR319a possède une séquence nucléotidique consistant en SEQ ID NO : 9, et ledit gène, impliqué dans le développement des parties végétatives ou reproductrices de la plante, est choisi parmi le groupe consistant en: *TCP3* (Accession n°AT1G53230.1) et *TCP4* (Accession n°AT3G15030.1).

Dans un mode de réalisation, l'invention concerne un procédé tel que défini ci-dessus, dans lequel ledit miPEP est le miPEP319a, en particulier, dans lequel ledit miPEP319a possède une séquence d'acides aminés consistant en SEQ ID NO : 10.

Dans un mode de réalisation, l'invention concerne un procédé tel que défini ci-dessus, dans lequel ladite plante est une plante crucifère telle qu'*Arabidopsis thaliana,* une plante légumineuse telle que *Glycine max* (soja), *Medicago truncatula* et *Medicago sativa* (luzerne) ou une plante solanacée telle que *Nicotiana benthamiana* (tabac), *Solanum tuberosum* (pomme de terre), *Solanum lycopersicum* (tomate) ou *Solanum melongena* (aubergine).

Dans un mode de réalisation, l'invention concerne un procédé tel que défini ci-dessus, dans lequel ladite plante est une plante crucifère.

Dans un mode de réalisation, l'invention concerne un procédé tel que défini ci-dessus, dans lequel ladite plante est *Arabidopsis thaliana.*
Dans un mode de réalisation, l'invention concerne un procédé tel que défini ci-dessus, dans lequel ladite plante est une plante crucifère et le miR est le miR164a.

Dans un mode de réalisation, l'invention concerne un procédé tel que défini ci-dessus, dans lequel ladite plante est une plante crucifère et le miR est le miR165a.

Dans un mode de réalisation, l'invention concerne un procédé tel que défini ci-dessus, dans lequel ladite plante est une plante crucifère et le miR est le miR319a.

Dans un mode de réalisation, l'invention concerne un procédé tel que défini ci-dessus, pour favoriser la croissance d'une plante *Arabidopsis thaliana,* dans lequel le miPEP164a est introduit par voie exogène dans ladite plante *Arabidopsis thaliana,* ledit miPEP164a étant également naturellement présent dans ladite plante *Arabidopsis thaliana,*
ledit miPEP164a introduit par voie exogène étant un peptide comprenant ou consistant en une séquence identique à celle dudit miPEP164a naturellement présent, lequel miPEP164a naturellement présent est un peptide de 3 à 100, notamment de 4 à 100 acides aminés, dont la séquence est codée par un cadre ouvert de lecture situé en 5' sur le transcrit primaire du miR164a,
ledit miPEP164a étant capable d'augmenter l'accumulation dudit miR164a, lequel miR164a régule l'expression d'au moins un gène impliqué dans le développement des parties végétatives ou reproductrices *d'Arabidopsis thaliana,*
la somme de la quantité dudit miPEP164a introduit par voie exogène et de celle dudit miPEP164a naturellement présent étant strictement supérieure à la quantité dudit miPEP164a naturellement présent.

Dans un mode de réalisation, l'invention concerne un procédé tel que défini ci-dessus, pour favoriser la croissance d'une plante *Arabidopsis thaliana,* dans lequel le miPEP165a est introduit par voie exogène dans ladite plante *Arabidopsis thaliana,* ledit miPEP165a étant également naturellement présent dans ladite plante *Arabidopsis thaliana,*
ledit miPEP165a introduit par voie exogène étant un peptide comprenant ou consistant en une séquence identique à celle dudit miPEP165a naturellement présent, lequel miPEP165a naturellement présent est un peptide de 3 à 100 acides aminés, notamment de 4 à 100 acides aminés dont la séquence est codée par un cadre ouvert de lecture situé en 5' sur le transcrit primaire du miR165a,
ledit miPEP165a étant capable d'augmenter l'accumulation dudit miR165a, lequel miR165a régule l'expression d'au moins un gène impliqué dans le développement des parties végétatives ou reproductrices *d'Arabidopsis thaliana,*
la somme de la quantité dudit miPEP165a introduit par voie exogène et de celle dudit miPEP165a naturellement présent étant strictement supérieure à la quantité dudit miPEP165a naturellement présent.

Dans un mode de réalisation, l'invention concerne un procédé tel que défini ci-dessus, pour favoriser la croissance d'une plante *Arabidopsis thaliana,* dans lequel le miPEP319a est introduit par voie exogène dans ladite plante *Arabidopsis thaliana,* ledit miPEP319a étant également naturellement présent dans ladite plante *Arabidopsis thaliana,*
ledit miPEP319a introduit par voie exogène étant un peptide comprenant ou consistant en une séquence identique à celle dudit miPEP319a naturellement présent, lequel miPEP319a naturellement présent est un peptide de 3 à 100 acides aminés, notamment de 4 à 100 acides aminés dont la séquence est codée par un cadre ouvert de lecture situé en 5' sur le transcrit primaire du miR319a,
ledit miPEP319a étant capable d'augmenter l'accumulation dudit miR319a, lequel miR319a régule l'expression d'au moins un gène impliqué dans le développement des parties végétatives ou reproductrices *d'Arabidopsis thaliana,*
la somme de la quantité dudit miPEP319a introduit par voie exogène et de celle dudit miPEP319a naturellement présent étant strictement supérieure à la quantité dudit miPEP319a naturellement présent.

Dans un mode de réalisation, l'invention concerne un procédé tel que défini ci-dessus, dans lequel ledit miPEP est introduit par voie externe dans la plante, de préférence par arrosage, par pulvérisation ou par l'ajout d'un engrais, d'un terreau, d'un substrat de culture ou d'un support en contact avec la plante.

Dans un mode de réalisation, l'invention concerne un procédé tel que défini ci-dessus, dans lequel ledit miPEP est introduit par voie externe dans la graine ou la semence, de préférence par arrosage, par pulvérisation ou par l'ajout d'un engrais, d'un terreau, d'un substrat de culture ou d'un support en contact avec la graine ou la semence.

Dans un mode de réalisation, l'invention concerne le procédé tel que défini ci-dessus, dans lequel ledit miPEP est utilisé pour traiter la plante sous forme de graine ou de semence.

Dans un mode de réalisation, l'invention concerne un procédé tel que défini ci-dessus, dans lequel ledit miPEP est administré à la plante sous la forme d'une composition comprenant 10⁻⁹ M à 10⁻⁴ M dudit miPEP, en particulier 10⁻⁹, 10⁻⁸, 10⁻⁷, 10⁻⁶, 10⁻⁵ ou 10⁻⁴ M dudit miPEP.

Dans un mode de réalisation, l'invention concerne un procédé tel que défini ci-dessus, dans lequel ledit miPEP est introduit dans la plante par le biais d'un acide nucléique codant ledit miPEP, ledit acide nucléique étant introduit dans la plante.

Dans un mode de réalisation, l'invention concerne un procédé tel que défini ci-dessus, dans laquelle la taille de la tige est augmentée chez la plante dans laquelle a été introduit ledit miPEP par rapport à la taille de la tige d'une plante identique et de même âge dans laquelle aucun miPEP n'a été introduit, ou bien par rapport à la taille de la tige d'une plante identique et de même âge dans laquelle ledit miPEP n'a pas été introduit.

Dans un mode de réalisation, l'invention concerne un procédé tel que défini ci-dessus, dans laquelle le nombre de feuilles est augmenté chez la plante dans laquelle a été introduit ledit miPEP par rapport au nombre de feuilles d'une plante identique et de même âge dans laquelle aucun miPEP n'a été introduit, ou bien par rapport au nombre de feuilles d'une plante identique et de même âge dans laquelle ledit miPEP n'a pas été introduit.

Dans un mode de réalisation, l'invention concerne un procédé tel que défini ci-dessus, dans laquelle la taille des feuilles est augmentée chez la plante dans laquelle a été introduit ledit miPEP par rapport à la taille des feuilles d'une plante identique et de même âge dans laquelle aucun miPEP n'a été introduit, ou bien par rapport à la taille des feuilles d'une plante identique et de même âge dans laquelle ledit miPEP n'a pas été introduit.

Dans un mode de réalisation, l'invention concerne un procédé tel que défini ci-dessus, dans laquelle le nombre de racines est augmenté chez la plante dans laquelle a été introduit ledit miPEP par rapport au nombre de racines d'une plante identique et de même âge dans laquelle aucun miPEP n'a été introduit, ou bien par rapport au nombre de racines d'une plante identique et de même âge dans laquelle ledit miPEP n'a pas été introduit.

Dans un mode de réalisation, l'invention concerne un procédé tel que défini ci-dessus, dans laquelle la longueur des racines est augmentée chez la plante dans laquelle a été introduit ledit miPEP par rapport à la longueur des racines d'une plante identique et de même âge dans laquelle aucun miPEP n'a été introduit, ou bien par rapport à la longueur des racines d'une plante identique et de même âge dans laquelle ledit miPEP n'a pas été introduit.

Dans un mode de réalisation, l'invention concerne un procédé tel que défini ci-dessus, dans laquelle le nombre de fleurs est augmenté chez la plante dans laquelle a été introduit ledit miPEP par rapport au nombre de fleurs d'une plante identique et de même âge dans laquelle aucun miPEP n'a été introduit, ou bien par rapport au nombre de fleurs d'une plante identique et de même âge dans laquelle ledit miPEP n'a pas été introduit.

Dans un mode de réalisation, l'invention concerne un procédé tel que défini ci-dessus, dans laquelle la date de floraison est avancée chez la plante dans laquelle a été introduit ledit miPEP par rapport à la date de floraison d'une plante identique et de même âge dans laquelle aucun miPEP n'a été introduit, ou bien par rapport à la date de floraison d'une plante identique et de même âge dans laquelle ledit miPEP n'a pas été introduit.

Dans un mode de réalisation, l'invention concerne un procédé tel que défini ci-dessus, dans laquelle la taille de la hampe florale est augmentée chez la plante dans laquelle a été introduit ledit miPEP par rapport à la taille de la hampe florale d'une plante identique et de même âge dans laquelle aucun miPEP n'a été introduit, ou bien par rapport à la taille de la hampe florale d'une plante identique et de même âge dans laquelle ledit miPEP n'a pas été introduit.

Dans un autre aspect, l'invention concerne une plante dans laquelle a été introduit un miPEP selon l'utilisation ou le procédé pour favoriser la croissance d'une plante décrits ci-dessus.

Dans un autre aspect, l'invention concerne un procédé de production d'une plante transgénique comprenant:
**a)** une étape d'introduction d'un acide nucléique codant un miPEP de 3 à 100 acides aminés, notamment de 4 à 100 acides aminés dans une plante, ou dans au moins une cellule de ladite plante, dans des conditions permettant l'expression dudit miPEP,
   ledit miPEP étant également naturellement présent dans ladite plante, ledit miPEP naturellement présent étant un peptide dont la séquence est codée par un cadre ouvert de lecture situé en 5' sur le transcrit primaire d'un miR, ledit miPEP étant capable de moduler l'accumulation dudit miR dans la plante, lequel miR régule l'expression d'au moins un gène impliqué dans le développement des parties végétatives ou reproductrices de la plante, notamment les racines, la tige, les feuilles ou les fleurs, et
**b)** une étape de culture de la plante, ou d'au moins une cellule de ladite plante, obtenues à l'étape a) dans des conditions permettant l'obtention d'une plante transgénique.

Dans un mode de réalisation, l'invention concerne un procédé de production d'une plante transgénique tel que défini ci-dessus, dans lequel ladite plante transgénique obtenue à l'étape b) a une croissance améliorée par rapport à une plante identique dans laquelle ledit acide nucléique n'a pas été introduit.

Dans un mode de réalisation, l'invention concerne un procédé de production d'une plante transgénique tel que défini ci-dessus, dans lequel l'étape a) est réalisée à l'aide d'un vecteur contenant ledit acide nucléique, de préférence un plasmide.

Dans un mode de réalisation, l'invention concerne un procédé de production d'une plante transgénique tel que défini ci-dessus, dans lequel ledit acide nucléique ne comprend pas la séquence complète dudit miR.

Dans un mode de réalisation, l'invention concerne un procédé de production d'une plante transgénique tel que défini ci-dessus, dans lequel l'expression dudit acide nucléique de l'étape a) est placée sous le contrôle d'un promoteur fort, de préférence un promoteur fort constitutif tel que le promoteur 35S.

Dans un mode de réalisation, l'invention concerne un procédé de production d'une plante transgénique tel que défini ci-dessus, dans lequel ledit gène, impliqué dans le développement des parties végétatives ou reproductrices de la plante, est choisi parmi le groupe consistant en: *NAC1* (Accession n°AT1G56010.1), *NAC4* (Accession n° ATSG07680.1), *NAC5* (Accession n° AT5G61430.1), *CUC1* (Accession n° AT3G15170.1) et *CUC2* (Accession n° AT5G53950.1).

Dans un mode de réalisation, l'invention concerne un procédé de production d'une plante transgénique tel que défini ci-dessus, dans lequel ledit miARN est le miR164a, en particulier, dans lequel ledit miR164a possède une séquence nucléotidique consistant en SEQ ID NO: 1.

Dans un mode de réalisation, l'invention concerne un procédé de production d'une plante transgénique tel que défini ci-dessus, dans lequel ledit miARN est le miR164a, en particulier, dans lequel ledit miR164a possède une séquence nucléotidique consistant en SEQ ID NO : 1, et ledit gène, impliqué dans le développement des parties végétatives ou reproductrices de la plante, est choisi parmi le groupe consistant en : *NAC1* (Accession n°AT1G56010.1), *NAC4* (Accession n° AT5G07680.1), *NAC5* (Accession n° AT5G61430.1), *CUC1* (Accession n° AT3G15170.1) et *CUC2* (Accession n° AT5G53950.1).

Dans un mode de réalisation, l'invention concerne un procédé de production d'une plante transgénique tel que défini ci-dessus, dans lequel ledit miPEP est le miPEP164a, en particulier, dans lequel ledit miPEP164a possède une séquence d'acides aminés consistant en SEQ ID NO : 2.

Dans un mode de réalisation, l'invention concerne un procédé de production d'une plante transgénique tel que défini ci-dessus, dans lequel ledit acide nucléique introduit à l'étape a) comprend une séquence nucléotidique consistant en SEQ ID NO : 3.

Dans un mode de réalisation, l'invention concerne un procédé de production d'une plante transgénique tel que défini ci-dessus, dans lequel ledit gène, impliqué dans le développement des parties végétatives ou reproductrices de la plante, est choisi parmi le groupe consistant en: *REVOLUTA* (Accession n° AT5G60690), *PHABULOSA* (Accession n° AT2G34710), *PHAVOLUTA* (Accession n° AT1G30490), *ATHB-8* (Accession n° AT4G32880) et *ATHB-15* (Accession n° AT1G52150).

Dans un mode de réalisation, l'invention concerne un procédé de production d'une plante transgénique tel que défini ci-dessus, dans lequel ledit miARN est le miR165a, en particulier, dans lequel ledit miR165a possède une séquence nucléotidique consistant en SEQ ID NO : 5.

Dans un mode de réalisation, l'invention concerne un procédé de production d'une plante transgénique tel que défini ci-dessus, dans lequel ledit miARN est le miR165a, en particulier, dans lequel ledit miR165a possède une séquence nucléotidique consistant en SEQ ID NO : 5, et ledit gène, impliqué dans le développement des parties végétatives ou reproductrices de la plante, est choisi parmi le groupe consistant en: *REVOLUTA* (Accession n° AT5G60690), *PHABULOSA* (Accession n° AT2G34710), *PHAVOLUTA* (Accession n° AT1G30490), *ATHB-8* (Accession n° AT4G32880) et *ATHB-15* (Accession n° AT1G52150).

Dans un mode de réalisation, l'invention concerne un procédé de production d'une plante transgénique tel que défini ci-dessus, dans lequel ledit miPEP est le miPEP165a, en particulier, dans lequel ledit miPEP165a possède une séquence d'acides aminés consistant en SEQ ID NO : 6.

Dans un mode de réalisation, l'invention concerne un procédé de production d'une plante transgénique tel que défini ci-dessus, dans lequel ledit acide nucléique introduit à l'étape a) comprend une séquence nucléotidique consistant en SEQ ID NO : 7.

Dans un mode de réalisation, l'invention concerne un procédé de production d'une plante transgénique tel que défini ci-dessus, dans lequel ledit gène, impliqué dans le développement des parties végétatives ou reproductrices de la plante, est choisi parmi le groupe consistant en: *TCP3* (Accession n°AT1G53230.1) et *TCP4* (Accession n°AT3G15030.1).

Dans un mode de réalisation, l'invention concerne un procédé de production d'une plante transgénique tel que défini ci-dessus, dans lequel ledit miARN est le miR319a, en particulier, dans lequel ledit miR319a possède une séquence nucléotidique consistant en SEQ ID NO : 9.

Dans un mode de réalisation, l'invention concerne un procédé de production d'une plante transgénique tel que défini ci-dessus, dans lequel ledit miARN est le miR319a, en particulier, dans lequel ledit miR319a possède une séquence nucléotidique consistant en SEQ ID NO : 9, et ledit gène, impliqué dans le développement des parties végétatives ou reproductrices de la plante, est choisi parmi le groupe consistant en: *TCP3* (Accession n°AT1G53230.1) et *TCP4* (Accession n°AT3G15030.1).

Dans un mode de réalisation, l'invention concerne un procédé de production d'une plante transgénique tel que défini ci-dessus, dans lequel ledit miPEP est le miPEP319a, en particulier, dans lequel ledit miPEP319a possède une séquence d'acides aminés consistant en SEQ ID NO : 10.

Dans un mode de réalisation, l'invention concerne un procédé de production d'une plante transgénique tel que défini ci-dessus, dans lequel ledit acide nucléique introduit à l'étape a) comprend une séquence nucléotidique consistant en SEQ ID NO : 11.

Dans un mode de réalisation, l'invention concerne un procédé de production d'une plante transgénique tel que défini ci-dessus, dans lequel ladite plante transgénique est une plante crucifère telle *qu'Arabidopsis thaliana,* une plante légumineuse telle que *Glycine max* (soja), *Medicago truncatula* et *Medicago sativa* (luzerne) ou une plante solanacée telle que *Nicotiana benthamiana* (tabac), *Solanum tuberosum* (pomme de terre), *Solanum lycopersicum* (tomate) ou *Solanum melongena* (aubergine).

Dans un mode de réalisation, l'invention concerne un procédé de production d'une plante transgénique tel que défini ci-dessus, dans lequel ladite plante transgénique est une plante crucifère.

Dans un mode de réalisation, l'invention concerne un procédé de production d'une plante transgénique tel que défini ci-dessus, dans lequel ladite plante transgénique est *Arabidopsis thaliana.*

Dans un mode de réalisation, l'invention concerne un procédé de production d'une plante transgénique tel que défini ci-dessus, comprenant :
**a)** une étape d'introduction d'un acide nucléique contenant la séquence nucléotidique SEQ ID NO : 3, codant le miPEP164a consistant en la séquence d'acides aminés SEQ ID NO : 2, dans une plante *Arabidopsis thaliana,* ou dans au moins une cellule de ladite plante *Arabidopsis thaliana,* dans des conditions permettant l'expression du miPEP164a,
   ledit miPEP164a étant également naturellement présent dans ladite plante *Arabidopsis thaliana,* ledit miPEP naturellement présent étant un peptide dont la séquence est codée par un cadre ouvert de lecture situé en 5' sur le transcrit primaire du miR164a, ledit miPEP164a étant capable de moduler l'accumulation dudit miR164, lequel miR164a régule l'expression d'au moins un gène impliqué dans le développement des parties végétatives ou reproductrices *d'Arabidopsis thaliana,* et
**b)** une étape de culture de la plante, ou d'au moins une cellule de ladite plante, obtenues à l'étape a) dans des conditions permettant l'obtention d'une plante *Arabidopsis thaliana* transgénique.

Dans un mode de réalisation, l'invention concerne un procédé de production d'une plante transgénique tel que défini ci-dessus, comprenant :
**a)** une étape d'introduction d'un acide nucléique contenant la séquence nucléotidique SEQ ID NO : 7, codant le miPEP165a consistant en la séquence d'acides aminés SEQ ID NO : 6, dans une plante *Arabidopsis thaliana,* ou dans au moins une cellule de ladite plante *Arabidopsis thaliana,* dans des conditions permettant l'expression du miPEP165a,
   ledit miPEP165a étant également naturellement présent dans ladite plante *Arabidopsis thaliana,* ledit miPEP naturellement présent étant un peptide dont la séquence est codée par un cadre ouvert de lecture situé en 5' sur le transcrit primaire du miR165a, ledit miPEP165a étant capable de moduler l'accumulation dudit miR165a, lequel miR165a régule l'expression d'au moins un gène impliqué dans le développement des parties végétatives ou reproductrices *d'Arabidopsis thaliana,* et
**b)** une étape de culture de la plante, ou d'au moins une cellule de ladite plante, obtenues à l'étape a) dans des conditions permettant l'obtention d'une plante *Arabidopsis thaliana* transgénique.

Dans un mode de réalisation, l'invention concerne un procédé de production d'une plante transgénique tel que défini ci-dessus, comprenant :
**a)** une étape d'introduction d'un acide nucléique contenant la séquence nucléotidique SEQ ID NO: 11, codant le miPEP319a consistant en la séquence d'acides aminés SEQ ID NO : 10, dans une plante *Arabidopsis thaliana,* ou dans au moins une cellule de ladite plante *Arabidopsis thaliana,* dans des conditions permettant l'expression du miPEP319a,
   ledit miPEP319a étant également naturellement présent dans ladite plante *Arabidopsis thaliana,* ledit miPEP naturellement présent étant un peptide dont la séquence est codée par un cadre ouvert de lecture situé en 5' sur le transcrit primaire du miR319a, ledit miPEP319a étant capable de moduler l'accumulation dudit miR319a, lequel miR319a régule l'expression d'au moins un gène impliqué dans le développement des parties végétatives ou reproductrices *d'Arabidopsis thaliana,* et
**b)** une étape de culture de la plante, ou d'au moins une cellule de ladite plante, obtenues à l'étape a) dans des conditions permettant l'obtention d'une plante *Arabidopsis thaliana* transgénique.

Dans un mode de réalisation, l'invention concerne un procédé de production tel que défini ci-dessus, dans lequel ledit miPEP est introduit dans la plante par le biais d'un acide nucléique codant ledit miPEP, ledit acide nucléique étant introduit dans la plante.

Dans un mode de réalisation, l'invention concerne un procédé de production d'une plante transgénique tel que défini ci-dessus, dans laquelle la taille de la tige est augmentée chez la plante dans laquelle a été introduit ledit miPEP par rapport à la taille de la tige d'une plante identique et de même âge dans laquelle aucun miPEP n'a été introduit, ou bien par rapport à la taille de la tige d'une plante identique et de même âge dans laquelle ledit miPEP n'a pas été introduit.

Dans un mode de réalisation, l'invention concerne un procédé de production d'une plante transgénique tel que défini ci-dessus, dans laquelle le nombre de feuilles est augmenté chez la plante dans laquelle a été introduit ledit miPEP par rapport au nombre de feuilles d'une plante identique et de même âge dans laquelle aucun miPEP n'a été introduit, ou bien par rapport au nombre de feuilles d'une plante identique et de même âge dans laquelle ledit miPEP n'a pas été introduit.

Dans un mode de réalisation, l'invention concerne un procédé de production d'une plante transgénique tel que défini ci-dessus, dans laquelle la taille des feuilles est augmentée chez la plante dans laquelle a été introduit ledit miPEP par rapport à la taille des feuilles d'une plante identique et de même âge dans laquelle aucun miPEP n'a été introduit, ou bien par rapport à la taille des feuilles d'une plante identique et de même âge dans laquelle ledit miPEP n'a pas été introduit.

Dans un mode de réalisation, l'invention concerne un procédé de production d'une plante transgénique tel que défini ci-dessus, dans laquelle le nombre de racines est augmenté chez la plante dans laquelle a été introduit ledit miPEP par rapport au nombre de racines d'une plante identique et de même âge dans laquelle aucun miPEP n'a été introduit, ou bien par rapport au nombre de racines d'une plante identique et de même âge dans laquelle ledit miPEP n'a pas été introduit.

Dans un mode de réalisation, l'invention concerne un procédé de production d'une plante transgénique tel que défini ci-dessus, dans laquelle la longueur des racines est augmentée chez la plante dans laquelle a été introduit ledit miPEP par rapport à la longueur des racines d'une plante identique et de même âge dans laquelle aucun miPEP n'a été introduit, ou bien par rapport à la longueur des racines d'une plante identique et de même âge dans laquelle ledit miPEP n'a pas été introduit.

Dans un mode de réalisation, l'invention concerne un procédé de production d'une plante transgénique tel que défini ci-dessus, dans laquelle le nombre de fleurs est augmenté chez la plante dans laquelle a été introduit ledit miPEP par rapport au nombre de fleurs d'une plante identique et de même âge dans laquelle aucun miPEP n'a été introduit, ou bien par rapport au nombre de fleurs d'une plante identique et de même âge dans laquelle ledit miPEP n'a pas été introduit.

Dans un mode de réalisation, l'invention concerne un procédé de production d'une plante transgénique tel que défini ci-dessus, dans laquelle la date de floraison est avancée chez la plante dans laquelle a été introduit ledit miPEP par rapport à la date de floraison d'une plante identique et de même âge dans laquelle aucun miPEP n'a été introduit, ou bien par rapport à la date de floraison d'une plante identique et de même âge dans laquelle ledit miPEP n'a pas été introduit.

Dans un mode de réalisation, l'invention concerne un procédé de production d'une plante transgénique tel que défini ci-dessus, dans laquelle la taille de la hampe florale est augmentée chez la plante dans laquelle a été introduit ledit miPEP par rapport à la taille de la hampe florale d'une plante identique et de même âge dans laquelle aucun miPEP n'a été introduit, ou bien par rapport à la taille de la hampe florale d'une plante identique et de même âge dans laquelle ledit miPEP n'a pas été introduit.

Dans un aspect, l'invention concerne également une plante transgénique telle qu'obtenue par le procédé de production tel que défini ci-dessus.

Dans un autre aspect, l'invention concerne une plante dans laquelle a été introduit un miPEP selon l'utilisation ou le procédé pour favoriser le développement des parties végétatives ou reproductrices de la plante.

Dans un autre aspect, l'invention concerne une composition, en particulier une composition phytosanitaire, comprenant le miPEP164a en tant que substance active, ledit miPEP164a consistant de préférence en SEQ ID NO : 1.

Dans un autre aspect, l'invention concerne une composition, en particulier une composition phytosanitaire, comprenant le miPEP165a en tant que substance active, ledit miPEP165a consistant de préférence en SEQ ID NO : 5.

Dans un autre aspect, l'invention concerne une composition, en particulier une composition phytosanitaire, comprenant le miPEP319a en tant que substance active, ledit miPEP319a consistant de préférence en SEQ ID NO : 9.

Dans un autre aspect, l'invention concerne une composition telle que définie ci-dessus, dans laquelle ledit miPEP164a est à une concentration de 10⁻⁹ M à 10⁻⁴ M, en particulier 10⁻⁹, 10⁻⁸, 10⁻⁷, 10⁻⁶, 10⁻⁵ ou 10⁻⁴ M.

De préférence, une composition telle que définie ci-dessus a une concentration de 10⁻⁸ M à 10⁻⁵ M pour une application par arrosage ou par pulvérisation sur la plante.

De manière complémentaire, des compositions plus ou moins concentrées peuvent être envisagées pour traiter la plante avec le miPEP. Par exemple, et de manière non limitative, des compositions plus concentrées comprenant 10⁻¹ M à 10⁻³ M, notamment 10⁻² M de miPEP, peuvent être utilisées dans le cas où le miPEP introduit par voie exogène est administré à la plante par épandage.

Dans un autre aspect, l'invention concerne une composition telle que définie ci-dessus, comprenant de plus un excipient, un diluant ou un solvant.

Dans un mode de réalisation, l'invention concerne une composition telle que définie ci-dessus formulée de façon à former un enrobé.

Dans un autre aspect, l'invention concerne une composition comprenant en combinaison une quantité de semences d'une plante et une quantité d'un peptide dont la séquence comprend ou consiste en une séquence identique à celle d'un miPEP naturellement présent chez ladite plante.

Dans un mode de réalisation, l'invention concerne une composition comprenant en combinaison une quantité de semences d'une plante crucifère, notamment A. *thaliana,* et une quantité d'un peptide dont la séquence comprend ou consiste en une séquence identique à celle du miPEP164a.

Dans un mode de réalisation, l'invention concerne une composition comprenant en combinaison une quantité de semences d'une plante crucifère, notamment *A*. *thaliana,* et une quantité d'un peptide dont la séquence comprend ou consiste en une séquence identique à celle du miPEP165a.

Dans un mode de réalisation, l'invention concerne une composition comprenant en combinaison une quantité de semences d'une plante crucifère, notamment *A*. *thaliana,* et une quantité d'un peptide dont la séquence comprend ou consiste en une séquence identique à celle du miPEP319a.

Dans un autre aspect, l'invention concerne une composition telle que définie ci-dessus, comprenant de plus un excipient, un diluant ou un solvant.

Dans un mode de réalisation, l'invention concerne une composition telle que définie ci-dessus formulée de façon à former une semence enrobée.

L'enrobage peut être réalisé selon les procédés classiquement utilisées dans l'industrie agroalimentaire et peut être obtenu en utilisant un matériau apte à se désagréger dans un solvant ou dans la terre, tel qu'un liant ou de l'argile.

Selon l'invention, l'enrobage peut être utilisé pour par exemple conférer des propriétés particulières à une composition de miPEP, ou encore à une composition de semences en combinaison avec un miPEP.

Dans un autre aspect, l'invention concerne un protocole de production d'un peptide recombinant, dont la séquence comprend ou consiste en une séquence identique à celle d'un miPEP tel que défini ci-dessus, comprenant une étape de transformation d'un organisme avec un vecteur d'expression codant ledit peptide recombinant.

Dans un mode de réalisation, ledit organisme est choisi parmi le groupe comprenant les bactéries, les levures, les champignons (autre que levures), les cellules animales, les plantes et les animaux.

Dans un mode de réalisation, ledit organisme est *Escherichia coli.*

En particulier, l'invention concerne un protocole de production d'un peptide recombinant tel que défini ci-dessus, comprenant les étapes suivantes :
- l'acide nucléique codant ledit peptide recombinant est lié à un acide nucléique codant une étiquette, telle que la GST,
- le vecteur d'expression contenant ledit acide nucléique codant ledit peptide recombinant est introduit dans la bactérie *E. coli,*
- la bactérie *E. coli* contenant le vecteur d'expression est cultivée dans du milieu LB de préférence jusqu'à une DO comprise entre 0.2 et 0.4,
- la production du peptide recombinant est induite avec de l'IPTG, de préférence pendant 4 à 5 heures,
- les bactéries *E. coli* sont centrifugées et lysées,
- le surnageant est filtré,
- ledit peptide recombinant est purifié sur une colonne d'affinité glutathion sepharose,
- si nécessaire, cliver la GST avec une protéase.

Dans un autre aspect, la description concerne un anticorps reconnaissant spécifiquement le miPEP164a, en particulier ledit miPEP164a consistant en SEQ ID NO : 2.

Dans un autre aspect, la description concerne un anticorps reconnaissant spécifiquement le miPEP165a, en particulier ledit miPEP165a consistant en SEQ ID NO : 6.

Dans un autre aspect, la description concerne un anticorps reconnaissant spécifiquement le miPEP319a, en particulier ledit miPEP319a consistant en SEQ ID NO : 10.

Un tel anticorps peut être obtenu à partir d'un procédé connu de l'homme du métier, comme par exemple en injectant ledit miPEP164a à un animal non humain pour déclencher une réaction d'immunisation et la production d'anticorps par ledit animal.

Dans un autre aspect, la description concerne un procédé d'immunolocalisation du miPEP164a comprenant une étape de marquage d'un échantillon biologique d'une plante avec un anticorps reconnaissant spécifiquement ledit miPEP164a.

Dans un autre aspect, la description concerne un procédé d'immunolocalisation du miPEP165a comprenant une étape de marquage d'un échantillon biologique d'une plante avec un anticorps reconnaissant spécifiquement ledit miPEP165a.

Dans un autre aspect, la description concerne un procédé d'immunolocalisation du miPEP319a comprenant une étape de marquage d'un échantillon biologique d'une plante avec un anticorps reconnaissant spécifiquement ledit miPEP319a.

Les séquences du miPEP164a, de son cadre ouvert de lecture, du miR164a et des transcrits primaires du miR164a chez *Arabidopsis thaliana* sont indiquées dans le tableau 1.

**Tableau 1.**

| | | |
|---|---|---|
| **miR164a** | uggagaagcagggcacgugca | **SEQ ID NO : 1** |
| **miPEP164a** | | **SEQ ID NO : 2** |
| **miORF164a** | | **SEQ ID NO : 3** |
| **pri-miR164a** | | **SEQ ID NO : 4** |
| | | |

Les séquences du miPEP165a, de son cadre ouvert de lecture, du miR164a et des transcrits primaires du miR165a chez *Arabidopsis thaliana* sont indiquées dans le tableau 2.

**Tableau 2.**

| | | |
|---|---|---|
| **miR165a** | ucggaccaggcuucauccccc | **SEQ ID NO : 5** |
| **miPEP165a** | MRVKLFQLRGMLSGSRIL | **SEQ ID NO : 6** |
| **miORF165a** | | **SEQ ID NO : 7** |
| **pri-miR165a** | | **SEQ ID NO : 8** |

Les séquences du miPEP319a, de son cadre ouvert de lecture, du miR319a et des transcrits primaires du miR319a chez *Arabidopsis thaliana* sont indiquées dans le tableau 3.

**Tableau 3.**

| | | |
|---|---|---|
| **miR319a** | uuggacugaagggagcucccu | **SEQ ID NO : 9** |
| **miPEP319a** | | **SEQ ID NO : 10** |
| **miORF319a** | | **SEQ ID NO : 11** |
| **pri-miR319a** | | **SEQ ID NO : 12** |

*L'objet de la demande* FR 13 60727 *concerne des micropeptides (peptides codés par des microARNs ou « miPEPs ») et leur utilisation pour moduler l'expression de gènes.*

*Les microARNs (miRs) sont des petits ARNs non codants, d'environ 21 nucléotides après maturation, qui contrôlent l'expression de gènes cibles au niveau post-transcriptionnel, en dégradant l'ARNm cible ou en inhibant sa traduction. Les miRs sont rencontrés chez les plantes et les animaux.*

*Les gènes cibles sont souvent des gènes clés de processus développementaux. Ils codent par exemple des facteurs de transcription ou des protéines du protéasome.*

*La régulation de l'expression des miRs est très peu connue, mais on sait notamment que celle-ci fait intervenir, à l'instar de la plupart des gènes codants, une ARN polymerase II : cette enzyme produit un transcrit primaire, appelé « pri-miR », qui est ensuite maturé par un complexe protéique contenant notamment les enzymes type Dicer. Cette maturation conduit tout d'abord à la formation d'un précurseur de miR appelé « pre-miR », ayant une structure secondaire en forme tige-boucle contenant le miR et sa séquence miR** *complémentaire. Puis le précurseur est maturé, ce qui conduit à la formation d'un ARN double brin plus court contenant le miR et le miR***. Le miR est ensuite pris en charge par le complexe RISC qui clive l'ARNm du gène cible ou bien inhibe sa traduction.*

*Par ailleurs, il a été montré que la présence d'introns dans le transcrit primaire du microARN augmente l'expression du microARN mature (*Schwab et al., EMBO Rep., 14(7):615-21, 2013*). Cependant, du fait de difficultés expérimentales, les transcrits primaires de microARNs, ou pri-miRs, sont très peu étudiés.*

*Environ 50% des gènes eucaryotes possèdent, au sein de leur région 5'UTR (5' UnTranslated Region) en amont de la séquence codante, de petits cadres ouverts de lecture. Ces petits cadres ouverts de lecture (ou « uORFs » pour upstream ORFs) peuvent jouer un rôle de régulateur de la traduction, principalement en cis, en modulant la fixation et la vitesse des ribosomes sur l'ARNm, mais également en trans selon un mécanisme encore inconnu, par l'intermédiaire de peptides codés par lesdits uORFs (*Combier et al., Gene Dev, 22:1549-1559, 2008*). Par définition, les uORFS sont présents en amont de gènes codants.*

*Récemment, il a également été découvert de petits ORFs dans de longs ARN non codants intergéniques (lincRNAs) dont la fonction putative, si elle existe, n'est pas connue (*Ingolia et al., Cell, 147(4):789-802, 2011 *;* Guttman & Rinn, Nature, 482(7385):339-46, 2012*).*

*Toutefois, aucun exemple n'a encore été rapporté concernant l'existence d'ORFs codant des peptides au sein de microARNs non codants. Jusqu'à présent, les microARNs, et par extension leur transcrit primaire, ont toujours été considérés, de par leur mode d'action particulier, comme des ARNs régulateurs non codants ne produisant aucun peptide.*

*L'un des aspects de l'objet de la demande* FR 13 60727 *est de proposer des peptides capables de moduler l'expression des microARNs.*
*Un autre aspect de l'objet de la demande* FR 13 60727 *est de proposer un moyen permettant de moduler l'expression d'un ou plusieurs gènes cibles d'un microARN.*
*L'objet de la demande* FR 13 60727 *présente l'avantage de permettre un contrôle plus facile et plus efficace de l'expression de gènes ciblés par les microARNs, à l'aide d'un moyen autre que le microARN.*

*L'objet de la demande* FR 13 60727 *concerne ainsi un procédé de détection et d'identification d'un micropeptide (miPEP) codé par une séquence nucléotidique contenue dans la séquence du transcrit primaire d'un microARN, comprenant :*
- *a) une étape de détection d'un cadre ouvert de lecture de 15 à 303 nucléotides contenu dans la séquence du transcrit primaire dudit microARN, puis*
- *b) une étape de comparaison entre :*
   - *l'accumulation dudit microARN dans une cellule eucaryote déterminée exprimant ledit microARN,*
      *en présence d'un peptide codé par une séquence nucléotidique identique ou dégénérée par rapport à celle dudit cadre ouvert de lecture, ledit peptide étant présent dans la cellule indépendamment de la transcription du transcrit primaire dudit microARN, et*
   - *l'accumulation dudit microARN dans une cellule eucaryote de même type que la susdite cellule eucaryote déterminée exprimant ledit microARN, en absence dudit peptide,*
   *dans lequel, une modulation de l'accumulation dudit microARN en présence dudit peptide par rapport à l'accumulation dudit microARN en absence dudit peptide indique l'existence d'un micropeptide codé par ledit cadre ouvert de lecture.*

*Dans une première étape, le procédé de détection et d'identification d'un micropeptide consiste donc à détecter sur le transcrit primaire d'un microARN l'existence d'un cadre ouvert de lecture codant potentiellement un peptide.*

*La deuxième étape permet, quant à elle, de caractériser ledit peptide, c'est-à-dire de déterminer si ledit peptide correspond a un peptide réellement produit dans la cellule, en recherchant un effet dudit peptide sur l'accumulation dudit microARN.*

*Pour mettre en évidence un effet du peptide sur l'accumulation du microARN, une quantité importante de peptide est introduite dans une première cellule exprimant ledit microARN. L'accumulation du microARN dans cette première cellule est ensuite mesurée et comparée avec l'accumulation du microARN dans une deuxième cellule identique à la première, mais ne contenant pas ledit peptide.*

*L'observation d'une variation des quantités de microARN entre les cellules en présence et en absence du peptide indique ainsi (i) qu'il existe un peptide codé sur le transcrit primaire dudit microARN, (ii) que la séquence de ce peptide est codée par le cadre ouvert de lecture identifié sur le transcrit primaire dudit microARN, et (iii) que ledit peptide agit sur l'accumulation dudit microARN.*

*L'objet de la demande* FR 13 60727 *repose donc sur la double observation inattendue faite par les Inventeurs que d'une part, il existe des cadres ouverts de lecture susceptibles de coder des micropeptides présents sur les transcrits primaires de microARNs, et d'autre part que lesdits micropeptides sont capables de moduler l'accumulation desdits microARNs.*

*Dans la demande* FR 13 60727*, les termes « microARN », « microARN non codant » et « miR » sont équivalents et peuvent être utilisés l'un pour l'autre. Ils définissent des petites molécules d'ARN d'environ 21 nucléotides, qui ne sont pas traduites et ne conduisent pas à un peptide ou une protéine.*
*Cependant, sous cette forme mature, les microARNs assurent une fonction de régulation de certains gènes via des mécanismes post-transcriptionnels, comme par exemple par l'intermédiaire du complexe RISC.*

*Le transcrit primaire du microARN ou « pri-miR » correspond lui à la molécule d'ARN directement obtenue à partir de la transcription de la molécule d'ADN. Généralement, ce transcrit primaire subit une ou plusieurs modifications post-transcriptionnelles, qui entraînent par exemple une structure particulière de l'ARN ou un clivage de certaines parties de l'ARN par des phénomènes d'épissage, et qui conduisent à la forme précurseur du microARN ou « pre-miR », puis à la forme mature du microARN ou « miR ».*

*Les termes « micropeptides » et « miPEPs » (microRNA encoded PEPtides) sont équivalents et peuvent être utilisés l'un pour l'autre. Ils définissent un peptide qui est codé par un cadre ouvert de lecture présent sur le transcrit primaire d'un microARN, et qui est capable de moduler l'accumulation dudit microARN. Les micropeptides au sens de la demande* FR 13 60727 *ne doivent être entendus comme étant nécessairement des peptides de petite taille, dans la mesure où « micro » ne correspond pas à la taille du peptide.*

*Compte tenu de la dégénérescence du code génétique, un même micropeptide peut être codé par plusieurs séquences nucléotidiques. De telles séquences nucléotidiques, différentes entre elles d'au moins un nucléotide mais codant un même peptide, sont appelées « séquences dégénérées ».*

*Les termes « cadre ouvert de lecture » ou « ORF » (open reading frame) sont équivalents et peuvent être utilisés l'un pour l'autre. Ils correspondent à une séquence de nucléotides dans une molécule d'ADN ou d'ARN pouvant potentiellement coder un peptide ou une protéine: ledit cadre ouvert de lecture débute par un codon start, suivi d'une série de codons, et se termine par un codon stop.*
*Dans la demande* FR 13 60727*, les ORFs peuvent être dénommés de manière spécifique « miORFs » lorsque ceux-ci sont présents sur les transcrits primaires de microARN.*

*Dans la demande* FR 13 60727*, par « accumulation », on entend la production d'une molécule, telle qu'un microARN ou un micropeptide, dans la cellule.*
*Ainsi, la « modulation » de l'accumulation d'une molécule dans une cellule correspond à une modification de la quantité de cette molécule présente dans la cellule.*

*Dans un mode de réalisation, l'objet de la demande* FR 13 60727 *concerne un procédé de détection et d'identification d'un miPEP tel que défini ci-dessus, dans lequel la modulation de l'accumulation dudit microARN est une diminution ou une augmentation de l'accumulation dudit microARN, en particulier une augmentation.*

*Une « diminution de l'accumulation » correspond à une baisse de la quantité de ladite molécule dans la cellule.*
*A l'inverse, une « augmentation de l'accumulation » correspond à une hausse de la quantité de ladite molécule dans la cellule.*

*Dans un mode de réalisation avantageux, l'objet de la demande* FR 13 60727 *concerne un procédé de détection et d'identification d'un miPEP tel que défini ci-dessus, dans lequel la modulation de l'accumulation dudit microARN est une augmentation de l'accumulation dudit microARN.*

*Dans un mode de réalisation, l'objet de la demande* FR 13 60727 *concerne un procédé de détection et d'identification d'un miPEP tel que défini ci-dessus, dans lequel la présence dudit peptide dans la cellule résulte :*
- *de l'introduction dans la cellule d'un acide nucléique codant ledit peptide, ou*
- *de l'introduction dans la cellule dudit peptide*

*De manière à caractériser un miPEP, il est nécessaire de disposer d'un modèle cellulaire exprimant un microARN et dans lequel ledit peptide à tester est présent. Pour cela, il est possible d'introduire un peptide dans la cellule, soit en mettant la cellule en contact avec le dit peptide, soit en introduisant dans la cellule un acide nucléique codant ledit peptide, lequel acide nucléique sera alors traduit en peptide à l'intérieur de la cellule.*

*Dans un mode de réalisation, l'objet de la demande* FR 13 60727 *concerne un procédé de détection et d'identification d'un miPEP tel que défini ci-dessus, dans lequel le dit cadre ouvert de lecture de l'étape a) est contenu dans la partie 5' ou 3' dudit transcrit primaire du microARN, de préférence dans la partie 5'.*

*Les parties 5' ou 3' du transcrit primaire du microARN correspondent aux parties terminales de la molécule de l'ARN qui sont clivées au cours de la maturation du microARN.*

*Dans un mode de réalisation, l'objet de la demande* FR 13 60727 *concerne un procédé de détection et d'identification d'un miPEP tel que défini ci-dessus, dans lequel ledit microARN est présent dans une cellule végétale sauvage.*

*Dans la demande* FR 13 60727*, une cellule végétale sauvage correspond à une cellule végétale qui n'a pas été modifiée génétiquement par l'homme.*

*Dans un mode de réalisation, l'objet de la demande* FR 13 60727 *concerne un procédé de détection et d'identification d'un miPEP tel que défini ci-dessus, dans lequel ladite cellule eucaryote déterminée, et ladite cellule eucaryote de même type que la susdite cellule eucaryote déterminée, utilisées à l'étape b, sont des cellules végétales, de préférence des cellules de Medicago truncatula ou d'Arabidopsis thaliana.*

*Dans le procédé de détection et d'identification d'un micropeptide tel que défini ci-dessus, après avoir identifié un ORF susceptible de coder un peptide sur le transcrit primaire d'un microARN, il est nécessaire de disposer d'un modèle cellulaire possédant ledit microARN et ledit peptide, pour pouvoir démontrer un effet éventuel du peptide sur ledit microARN.*

*Deux options sont donc envisageables :*
- *le modèle cellulaire dans lequel a été identifié le miORF et celui dans lequel est mis en évidence l'effet du peptide sur le miR sont identiques, ou*
- *le modèle cellulaire dans lequel a été identifié le miORF et celui dans lequel est mis en évidence l'effet du peptide sur le miR sont différents.*

*Dans la première option, le modèle cellulaire utilisé pour observer un effet du peptide est le même que celui dans lequel le transcrit primaire dudit microARN a été isolé. Dans ce modèle cellulaire, les cellules eucaryotes déterminées contiennent naturellement ledit microARN et seul le peptide à tester doit être introduit dans ces cellules. Dans ce contexte, ledit microARN est qualifié « d'origine endogène » car celui-ci existe naturellement dans les cellules. Néanmoins, dans une cellule, d'autres copies d'un microARN d'origine endogène peuvent être ajoutées, comme par exemple en introduisant dans la cellule un vecteur codant ledit microARN d'origine endogène.*

*Dans la deuxième option, le modèle cellulaire utilisé pour observer un effet du peptide est différent de celui dans lequel le transcrit primaire dudit microARN a été isolé. Dans ce modèle cellulaire, les cellules eucaryotes déterminées ne contiennent ni le microARN, ni le peptide à tester. Ces deux éléments doivent donc être introduits dans ces cellules. Dans ce contexte, ledit microARN est qualifié « d'origine exogène » car celui-ci n'existe pas naturellement dans les cellules.*

*Dans un mode de réalisation, l'objet de la demande* FR 13 60727 *concerne un procédé de détection et d'identification d'un miPEP tel que défini ci-dessus, dans lequel ledit microARN est d'origine endogène dans ladite cellule eucaryote et dans ladite cellule eucaryote de même type que la susdite cellule eucaryote déterminée, utilisées à l'étape b).*

*Dans un mode de réalisation, l'objet de la demande* FR 13 60727 *concerne un procédé de détection et d'identification d'un miPEP tel que défini ci-dessus dans lequel ledit microARN est d'origine exogène dans ladite cellule eucaryote et dans ladite cellule eucaryote de même type que la susdite cellule eucaryote déterminée, utilisées à l'étape b), lesdites cellules eucaryotes contenant un vecteur permettant l'expression dudit microARN.*

*Dans un mode de réalisation, l'objet de la demande* FR 13 60727 *concerne un procédé de détection et d'identification d'un miPEP tel que défini ci-dessus, dans lequel l'accumulation dudit microARN est déterminée en mettant en œuvre une RT-PCR quantitative ou un Northern blot.*

*Dans un mode de réalisation, l'objet de la demande* FR 13 60727 *concerne un procédé de détection et d'identification d'un miPEP tel que défini ci-dessus, dans lequel l'accumulation dudit microARN est déterminée en mettant en œuvre une puce à ADN ou à ARN.*

*L'accumulation dudit microARN peut être déterminée à l'aide des techniques de biologie moléculaire permettant le dosage de molécules d'acides nucléiques spécifiques.*

*Dans un autre aspect, l'objet de la demande* FR 13 60727 *concerne également un procédé de détection et d'identification d'un microARN dont la séquence du transcrit primaire contient une séquence nucléotidique codant un miPEP,*
*comprenant :*
- *a) une étape de détection d'un cadre ouvert de lecture de 15 à 303 nucléotides contenu dans la séquence du transcrit primaire dudit microARN, puis*
- *b) une étape de comparaison entre :*
   - *l'accumulation dudit microARN dans une cellule eucaryote déterminée exprimant ledit microARN,*
      *en présence d'un peptide codé par une séquence nucléotidique identique ou dégénérée par rapport à celle dudit cadre ouvert de lecture, ledit peptide étant présent dans la cellule indépendamment de la transcription du transcrit primaire dudit microARN, et*
   - *l'accumulation dudit microARN dans une cellule eucaryote, de même type que la susdite cellule eucaryote déterminée exprimant ledit microARN, en absence dudit peptide,*
   *dans lequel, une modulation de l'accumulation dudit microARN en présence dudit peptide par rapport à l'accumulation dudit microARN en absence dudit peptide indique l'existence d'un microARN dont le transcrit primaire contient une séquence nucléotidique codant un micropeptide.*

*Dans un mode de réalisation, l'objet de la demande* FR 13 60727 *concerne un procédé de détection et d'identification d'un microARN tel que défini ci-dessus, dans lequel la modulation de l'accumulation dudit microARN est une diminution ou une augmentation de l'accumulation dudit microARN, en particulier une augmentation.*

*Dans un mode de réalisation, l'objet de la demande* FR 13 60727 *concerne un procédé de détection et d'identification d'un microARN tel que défini ci-dessus, dans lequel la présence dudit peptide dans la cellule résulte :*
- *de l'introduction dans la cellule d'un acide nucléique codant ledit peptide, ou*
- *de l'introduction dans la cellule de ledit peptide.*

*Dans un mode de réalisation, l'objet de la demande* FR 13 60727 *concerne un procédé de détection et d'identification d'un microARN tel que défini ci-dessus, dans lequel le dit cadre ouvert de lecture de l'étape a) est contenu dans la partie 5' ou 3' dudit transcrit primaire du microARN, de préférence dans la partie 5'.*

*Dans un mode de réalisation, l'objet de la demande* FR 13 60727 *concerne un procédé de détection et d'identification d'un microARN tel que défini ci-dessus, dans lequel ledit microARN est présent dans une cellule végétale sauvage.*

*Dans un mode de réalisation, l'objet de la demande* FR 13 60727 *concerne un procédé de détection et d'identification d'un microARN tel que défini ci-dessus, dans lequel ladite cellule eucaryote, et ladite cellule eucaryote de même type que la susdite cellule eucaryote déterminée, utilisées à l'étape b) sont des cellules végétales, de préférence des cellules de Medicago truncatula.*

*Dans un mode de réalisation, l'objet de la demande* FR 13 60727 *concerne un procédé de détection et d'identification d'un microARN tel que défini ci-dessus, dans lequel ledit microARN est d'origine endogène dans ladite cellule eucaryote et dans ladite cellule eucaryote de même type que la susdite cellule eucaryote déterminée, utilisées à l'étape b).*

*Dans un mode de réalisation, l'objet de la demande* FR 13 60727 *concerne un procédé de détection et d'identification d'un microARN tel que défini ci-dessus dans lequel ledit microARN est d'origine exogène dans ladite cellule eucaryote et dans ladite cellule eucaryote de même type que la susdite cellule eucaryote déterminée, utilisées à l'étape b), lesdites cellules eucaryotes contenant un vecteur permettant l'expression dudit microARN.*

*Dans un mode de réalisation, l'objet de la demande* FR 13 60727 *concerne un procédé de détection et d'identification d'un microARN tel que défini ci-dessus, dans lequel l'accumulation dudit microARN est déterminée en mettant en œuvre une RT-PCR quantitative ou un Northern blot.*

*Dans un mode de réalisation, l'objet de la demande* FR 13 60727 *concerne un procédé de détection et d'identification d'un microARN tel que défini ci-dessus, dans lequel l'accumulation dudit microARN est déterminée en mettant en œuvre une puce à ADN ou à ARN.*

*Dans un autre aspect, l'objet de la demande* FR 13 60727 *concerne un miPEP tel qu'obtenu par la mise en œuvre du procédé tel que défini ci-dessus.*

*Un autre aspect de l'objet de la demande* FR 13 60727 *concerne également un miPEP de 4 à 100 acides aminés, de préférence de 4 à 40 acides aminés, codé par une séquence nucléotidique contenue dans le transcrit primaire d'un microARN, ledit miPEP étant capable de moduler l'accumulation dudit microARN dans une cellule eucaryote.*

*Par ailleurs, il convient de noter que plusieurs miORFS peuvent être identifiés sur le transcrit primaire d'un microARN, indiquant qu'un transcrit primaire de microARN peut potentiellement coder plusieurs miPEPs.*

*Il convient également de noter que l'effet d'un miPEP est généralement spécifique d'un seul microARN, à savoir celui résultant du transcrit primaire codant ledit miPEP.*

*Dans un mode de réalisation, l'objet de la demande* FR 13 60727 *concerne un miPEP tel que défini ci-dessus, ladite séquence nucléotidique étant contenue dans la partie 5' ou 3' dudit transcrit primaire d'un microARN, de préférence dans la partie 5'.*

*Dans un mode de réalisation, l'objet de la demande* FR 13 60727 *concerne un miPEP tel que défini ci-dessus, ladite séquence nucléotidique correspondant au premier cadre ouvert de lecture présent sur ledit transcrit primaire d'un microARN.*

*Dans un mode de réalisation, l'objet de la demande* FR 13 60727 *concerne un miPEP tel que défini ci-dessus, ledit miPEP possédant un point isoélectrique basique, de préférence supérieur à 8.*

*Dans un autre aspect, l'objet de la demande* FR 13 60727 *concerne une molécule d'acide nucléique codant un miPEP tel que défini ci-dessus.*

*Dans autre aspect, l'objet de la demande* FR 13 60727 *concerne un vecteur comprenant au moins une molécule d'acide nucléique tel que définie ci-dessus.*

*Dans un autre aspect, l'objet de la demande* FR 13 60727 *concerne également l'utilisation d'au moins :*
- *un miPEP tel que défini ci-dessus,*
- *un acide nucléique codant ledit miPEP, ou*
- *un vecteur contenant ledit acide nucléique,*
*pour moduler l'expression d'au moins un gène dans une cellule eucaryote déterminée, ladite cellule eucaryote déterminée étant capable d'exprimer un microARN, dont le transcrit primaire contient au moins une séquence nucléotidique codant ledit au moins un miPEP et dont l'accumulation est modulée par ledit au moins un miPEP, l'expression dudit au moins un gène étant régulée par ledit microARN.*

*Dans un autre aspect, l'objet de la demande* FR 13 60727 *concerne également l'utilisation d'au moins :*
- *un miPEP de 4 à 100 acides aminés, de préférence de 4 à 40 acides aminés, codé par une séquence nucléotidique contenue dans le transcrit primaire d'un microARN, ledit miPEP étant capable de moduler l'accumulation dudit microARN dans une cellule eucaryote,*
- *un acide nucléique codant ledit miPEP, ou*
- *un vecteur contenant ledit acide nucléique,*
*pour moduler l'expression d'au moins un gène dans une cellule eucaryote déterminée, ladite cellule eucaryote déterminée étant capable d'exprimer un microARN, dont le transcrit primaire contient au moins une séquence nucléotidique codant ledit au moins un miPEP et dont l'accumulation est modulée par ledit au moins un miPEP, l'expression dudit au moins un gène étant régulée par ledit microARN.*

*L'objet de la demande* FR 13 60727 *repose sur l'observation surprenante faite par les Inventeurs qu'il est possible de moduler l'expression d'un ou plusieurs gènes cibles d'un même microARN en modulant l'accumulation dudit microARN à l'aide d'un miPEP.*

*Dans un mode de réalisation, l'objet de la demande* FR 13 60727 *concerne l'utilisation telle que définie ci-dessus dans laquelle ladite cellule eucaryote déterminée est une cellule végétale.*

*Dans un mode de réalisation, l'objet de la demande* FR 13 60727 *concerne l'utilisation telle que définie ci-dessus dans laquelle ledit microARN et ledit gène sont d'origine endogène dans ladite cellule eucaryote déterminée.*

*Dans un mode de réalisation, l'objet de la demande* FR 13 60727 *concerne l'utilisation telle que définie ci-dessus dans laquelle ledit microARN et ledit gène sont d'origine exogène dans ladite cellule eucaryote déterminée, ladite cellule eucaryote déterminée contenant au moins un vecteur permettant l'expression dudit microARN et dudit gène.*

*Dans la demande* FR 13 60727*, les expressions « d'origine endogène » et « d'origine exogène » sont utilisées pour distinguer lesdits microARNs et*/*ou les gènes d'espèces différentes, étant donné la conservation des séquences entre espèces.*
*Ainsi, le terme « d'origine endogène » indique que le microARN et*/*ou le gène peuvent être présents de manière naturelle dans la cellule en question. De manière artificielle, d'autres copies du microARN et*/*ou du gène d'origine endogène peuvent néanmoins être ajoutées dans la cellule en question, comme par exemple par clonage.*
*A l'inverse, le terme « d'origine exogène » indique que le microARN et*/*ou le gène ne sont jamais présents naturellement dans la cellule en question. Il s'agit d'un microARN et*/*ou d'un gène identifié dans un autre type cellulaire ou dans un organisme d'une autre espèce, ce microARN et*/*ou ce gène sont donc nécessairement introduits artificiellement dans la cellule en question.*

*Dans la demande* FR 13 60727*, une cellule transformée génétiquement peut donc contenir 2 groupes de microARNs et*/*ou de gènes potentiellement proches en termes de séquence, l'un d'origine endogène et l'autre d'origine exogène.*

*Dans un autre aspect, l'objet de la demande* FR 13 60727 *concerne un procédé de modulation de l'expression d'un gène régulée par un microARN dans une cellule eucaryote,*
*comprenant la mise en œuvre d'une étape d'accumulation d'un miPEP dans ladite cellule eucaryote,*
*ledit miPEP ayant :*
- *une taille de 4 à 100 acides aminés, de préférence 4 à 20 acides aminés, et*
- *une séquence peptidique identique à celle codée par une séquence nucléotidique contenue dans le transcrit primaire d'un microARN régulant l'expression dudit gène, et*
- *étant capable de moduler l'accumulation dudit microARN,*
*dans lequel, l'accumulation dudit miPEP dans ladite cellule eucaryote induit une modulation de l'expression dudit gène par rapport à l'expression dudit gène sans accumulation dudit miPEP.*

*Dans un mode de réalisation, l'objet de la demande* FR 13 60727 *concerne un procédé de modulation de l'expression d'un gène tel que défini ci-dessus, dans lequel l'accumulation dudit miPEP dans la cellule résulte :*
- *de l'introduction dans la cellule d'un acide nucléique codant ledit miPEP, ou*
- *de l'introduction dans la cellule dudit miPEP.*

*Dans un mode de réalisation, l'objet de la demande* FR 13 60727 *concerne un procédé de modulation de l'expression d'un gène tel que défini ci-dessus dans lequel ladite cellule eucaryote est une cellule végétale.*

*Dans un mode de réalisation, l'objet de la demande* FR 13 60727 *concerne un procédé de modulation de l'expression d'un gène tel que défini ci-dessus dans lequel ledit microARN et ledit gène sont d'origine endogène dans ladite cellule eucaryote.*

*Dans un mode de réalisation, l'objet de la demande* FR 13 60727 *concerne un procédé de modulation de l'expression d'un gène tel que défini ci-dessus dans lequel ledit microARN et ledit gène sont d'origine exogène dans ladite cellule eucaryote, ladite cellule eucaryote contenant au moins un vecteur permettant l'expression dudit microARN et dudit gène.*

*Dans un autre aspect, l'objet de la demande* FR 13 60727 *concerne une cellule eucaryote modifiée contenant un peptide identique à un miPEP tel que défini ci-dessus, lequel peptide est présent dans ladite cellule eucaryote indépendamment de la transcription du transcrit primaire du microARN portant la séquence nucléotidique codant ledit miPEP.*

*Dans la demande* FR 13 60727*, le terme « cellule eucaryote modifiée » signifie que ladite cellule eucaryote contient un miPEP introduit artificiellement dans la cellule, que ce soit en tant que peptide, ou par l'intermédiaire d'un vecteur codant ledit miPEP.*

*Dans un mode de réalisation, l'objet de la demande* FR 13 60727 *concerne une cellule eucaryote modifiée telle que définie ci-dessus, dans laquelle ledit microARN est d'origine endogène.*

*Dans un autre mode de réalisation, l'objet de la demande* FR 13 60727 *concerne une cellule eucaryote modifiée telle que définie ci-dessus dans laquelle ledit microARN est d'origine exogène, ladite cellule eucaryote modifiée contenant un vecteur permettant l'expression dudit microARN.*

*Dans un mode de réalisation, l'objet de la demande* FR 13 60727 *concerne une cellule eucaryote modifiée telle que définie ci-dessus, ladite cellule étant une cellule végétale. Dans un autre aspect, l'objet de la demande* FR 13 60727 *concerne une plante comprenant au moins une cellule eucaryote modifiée telle que définie ci-dessus.*

*Dans un autre aspect, l'objet de la demande* FR 13 60727 *concerne une composition comprenant au moins :*
- *un miPEP tel que défini ci-dessus,*
- *un acide nucléique codant ledit miPEP, ou*
- *un vecteur contenant ledit acide nucléique.*

*Dans un autre aspect, l'objet de la demande* FR 13 60727 *concerne une composition pesticide comprenant au moins :*
- *un miPEP tel que défini ci-dessus,*
- *un acide nucléique codant ledit miPEP, ou*
- *un vecteur contenant ledit acide nucléique.*

*Dans un autre aspect, l'objet de la demande* FR 13 60727 *concerne une composition phytopharmaceutique comprenant au moins :*
- *un miPEP tel que défini ci-dessus,*
- *un acide nucléique codant ledit miPEP, ou*
- *un vecteur contenant ledit acide nucléique.*

*Dans un autre aspect, l'objet de la demande* FR 13 60727 *concerne une composition élicitrice comprenant au moins :*
- *un miPEP tel que défini ci-dessus,*
- *un acide nucléique codant ledit miPEP, ou*
- *un vecteur contenant ledit acide nucléique.*

*Par « composition élicitrice », on désigne une composition capable de conférer à la plante une meilleure aptitude à la symbiose ou une meilleure résistance à différents stress, qu'ils soient de nature thermiques, hydriques ou chimiques.*
*A cet effet, l'objet de la demande* FR 13 60727 *concerne également des compositions agissant sur la croissance (inhibition de la croissance ou au contraire augmentation de la croissance) et la physiologie (meilleure aptitude à mycorhizer, noduler, meilleure tolérance à différents stress) de la plante.*

*Dans un autre aspect, l'objet de la demande* FR 13 60727 *concerne une composition herbicide comprenant au moins :*
- *un miPEP tel que défini ci-dessus,*
- *un acide nucléique codant ledit miPEP, ou*
- *un vecteur contenant ledit acide nucléique.*

*Dans un autre aspect, l'objet de la demande* FR 13 60727 *concerne une composition insecticide comprenant au moins :*
- *un miPEP tel que défini ci-dessus,*
- *un acide nucléique codant ledit miPEP, ou*
- *un vecteur contenant ledit acide nucléique.*

*Dans un autre aspect, l'objet de la demande* FR 13 60727 *concerne l'utilisation d'une composition telle que définie ci-dessus, en tant qu'herbicide pour éliminer les plantes ou ralentir leur croissance, de préférence en tant qu'herbicide spécifique d'une espèce ou d'un genre de plantes.*

*Dans un autre aspect, l'objet de la demande* FR 13 60727 *concerne l'utilisation d'une composition telle que définie ci-dessus, en tant qu'agent phytopharmaceutique,*
- *pour favoriser la croissance et*/*ou le développement des plantes, notamment pour la modulation des paramètres physiologiques d'une plante, en particulier la biomasse, la surface foliaire, la floraison, le calibre du fruit, la production et*/*ou la sélection de semence végétales, en particulier pour contrôler la parthénocarpie ou la monoecie d'une plante, ou pour la modification des paramètres physiologiques de semences végétales, en particulier la germination, l'implantation racinaire et la résistance au stress hydrique,*
- *ou pour prévenir ou traiter les maladies des plantes,*
*en particulier pour favoriser la résistance aux maladies infectieuses.*

*Dans un autre aspect, l'objet de la demande* FR 13 60727 *concerne l'utilisation d'une composition telle que définie ci-dessus, pour moduler les paramètres physiologiques d'une plante, en particulier la biomasse, la surface foliaire, ou le calibre du fruit.*

*Dans un mode de réalisation, l'objet de la demande* FR 13 60727 *concerne l'utilisation d'une composition telle que définie ci-dessus, pour l'éclaircissage de vergers afin d'augmenter le calibre des fruits.*

*Dans un mode de réalisation, l'objet de la demande* FR 13 60727 *concerne l'utilisation d'une composition telle que définie ci-dessus, pour la production et*/*ou la sélection de semences végétales, ladite composition étant utilisée pour contrôler la parthénocarpie ou la monœcie d'une plante.*

*Dans un mode de réalisation, l'objet de la demande* FR 13 60727 *concerne l'utilisation d'une composition telle que définie ci-dessus, ladite composition étant administrée à ladite plante par la voie foliaire ou par la voie racinaire.*

*Dans un mode de réalisation, l'objet de la demande* FR 13 60727 *concerne l'utilisation d'une composition telle que définie ci-dessus, pour la production et*/*ou la sélection de semences végétales.*

*Dans un mode de réalisation, l'objet de la demande* FR 13 60727 *concerne l'utilisation d'une composition telle que définie ci-dessus, dans laquelle ladite composition est utilisée pour modifier les paramètres physiologiques desdites semences végétales, en particulier l'implantation racinaire, la germination et la résistance au stress hydrique.*

*Dans un mode de réalisation, l'objet de la demande* FR 13 60727 *concerne l'utilisation d'une composition telle que définie ci-dessus, dans laquelle ladite composition est appliquée par enrobage ou pelliculage sur lesdites semences végétales.*
*Dans un autre aspect, l'objet de la demande* FR 13 60727 *concerne l'utilisation d'une composition telle que définie ci-dessus, en tant que pesticide, pour éliminer les organismes nuisibles des plantes ou susceptibles d'être classés comme tels,*
*notamment en tant qu'insecticide, arachnicide, limacide ou rodonticide.*
*Dans un mode de réalisation, l'objet de la demande* FR 13 60727 *concerne l'utilisation d'une composition telle que définie ci-dessus, en tant qu'insecticide.*

*Dans un mode de réalisation, l'objet de la demande* FR 13 60727 *concerne l'utilisation d'une composition telle que définie ci-dessus, pour éliminer les insectes ravageurs.*

*Dans un mode de réalisation, l'objet de la demande* FR 13 60727 *concerne l'utilisation d'une composition telle que définie ci-dessus, pour éliminer les espèces animales classées nuisibles ou susceptibles d'être classées comme telles, en particulier les muridae, notamment le rat.*

*Dans un autre aspect, l'objet de la demande* FR 13 60727 *concerne l'utilisation d'une composition telle que définie ci-dessus, dans laquelle ladite composition est appliquée sur une plante pour la protéger d'insectes ravageurs.*

Les figures et les exemples suivants illustreront mieux l'invention, sans pour autant en limiter sa portée.

### LEGENDES DES FIGURES

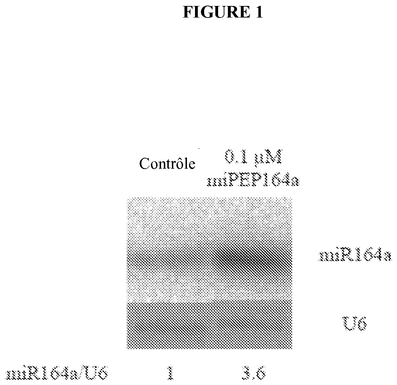

**FIGURE 1****. Effets d'un traitement avec le miPEP164a sur l'expression du miR164a chez *A. thaliana.***
   Les photographies représentent les résultats d'une analyse par Northern blot de l'accumulation du miR164a dans des racines traitées avec de l'eau (contrôle, photographie de gauche) ou avec 0,1 µM d'un peptide synthétique, ayant une séquence identique à celle du miPEP164a, dissout dans de l'eau (0,1 µM miPEP164a). L'ARN U6 est utilisé comme témoin de charge permettant de quantifier la quantité de miR164a.
   Cette expérience a été répétée 4 fois de manière indépendante et a abouti à des résultats similaires.
   Le traitement des pousses d'*A*. *thaliana* avec 0,1 µM de miPEP164a entraîne une augmentation de l'accumulation du miR164a.

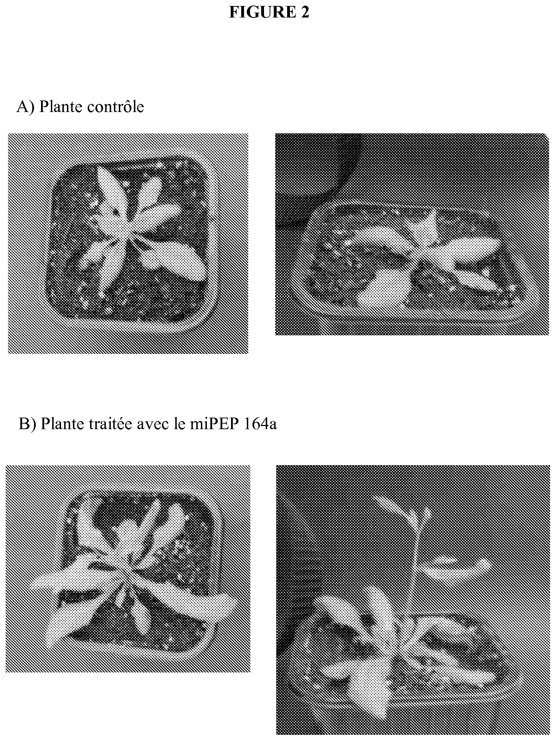

**FIGURE 2****. Effets d'un traitement avec le miPEP164a sur la croissance d'*Arabidopsis thaliana.***
   Les photographies présentent deux plantes (vues de dessus et vues de côté) après 3 semaines de croissance : une plante contrôle arrosée et vaporisée avec de l'eau (A) et une plante arrosée et vaporisée avec une composition de 0,1 µM de peptide synthétique dont la séquence est identique miPEP164a (B). L'arrosage des plantes d'*Arabidopsis thaliana avec* le miPEP164a augmente significativement la croissance de la plante.

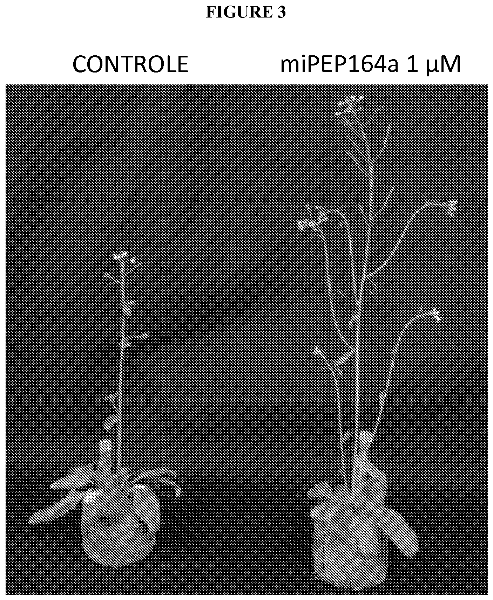

**FIGURE 3****. Effets d'un traitement avec le miPEP164a sur la date de floraison chez *Arabidopsis thaliana***
   Les photographies présentent deux plantes après 39 jours de croissance : une plante contrôle arrosée et vaporisée avec de l'eau (photographie de gauche) et une plante arrosée et vaporisée avec une composition de 1 µM de peptide synthétique dont la séquence est identique miPEP164a (photographie de droite). L'arrosage des plantes d'*Arabidopsis thaliana avec* le miPEP164a augmente significativement la croissance et le nombre de fleurs de la plante.

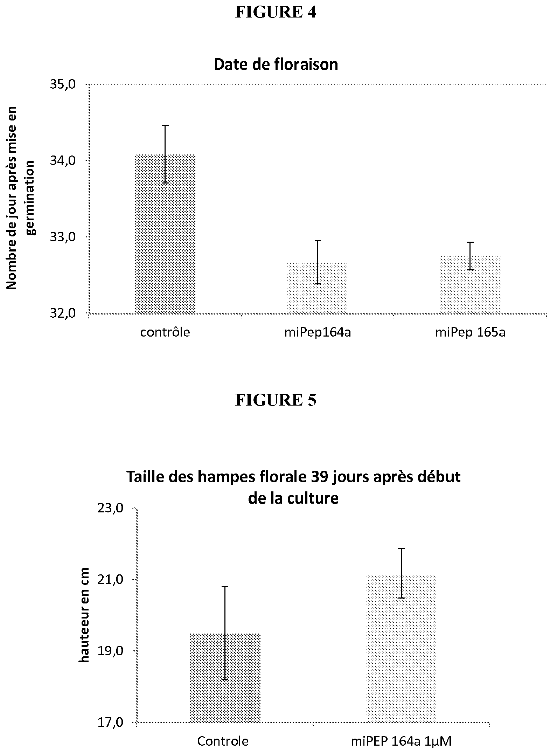

**FIGURE 4****. Effets d'un traitement avec le miPEP164a sur la date de floraison chez *Arabidopsis thaliana***
   L'axe des ordonnées indique le nombre de jours après mise en germination à la date de floraison chez une plante traitée avec de l'eau (barre de gauche) ou avec une composition contenant 1 µM de miPEP164a (barre du milieu) et 1 µM de miPEP165a (barre de droite). La barre d'erreur correspond à l'erreur standard à la moyenne (nombre d'individus = 12 plantes contrôles, 12 plantes traitées).
   Cette expérience a été répétée de manière indépendante et a abouti à des résultats similaires.
**FIGURE 5****. Effets d'un traitement avec le miPEP164a sur la taille des hampes florales chez *Arabidopsis thaliana***
   L'axe des ordonnées indique la hauteur de la hampe florale chez une plante traitée avec de l'eau (barre de gauche) ou avec une composition contenant 0,1 µM de miPEP164a (barre de droite).
   La barre d'erreur correspond à l'erreur standard à la moyenne (nombre d'individus = 12 plantes contrôles, 12 plantes traitées).

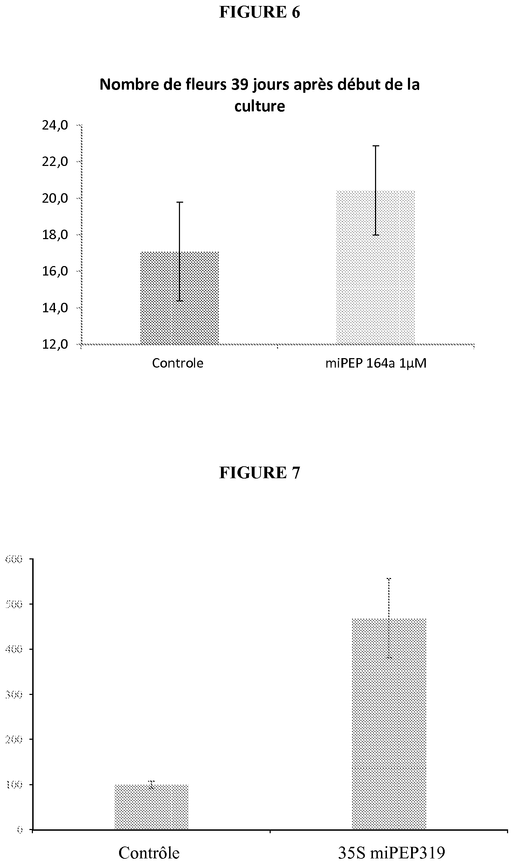

**FIGURE 6****. Effets d'un traitement avec le miPEP164a sur le nombre de fleurs chez *Arabidopsis thaliana***
   L'axe des ordonnées indique le nombre de fleurs après 39 jours de culture chez une plante traitée avec de l'eau (barre de gauche) ou avec une composition contenant 0,1 µM de miPEP164a (barre de droite).
   La barre d'erreur correspond à l'erreur standard à la moyenne (nombre d'individus = 12 plantes contrôles, 12 plantes traitées).
**FIGURE 7****. Effets de la surexpression du AtmiPEP319a sur l'expression du AtmiR319a chez *A. thaliana.***
   L'axe des ordonnées indique l'expression relative du AtmiR319a chez une plante témoin (colonne de gauche) ou chez une plante dans laquelle est surexprimé AtmiPEP319a (colonne de droite). La barre d'erreur correspond à l'erreur standard à la moyenne (nombre d'individus = 10). La surexpression de AtmiPEP319a induit une augmentation de l'accumulation de Atmir319a.

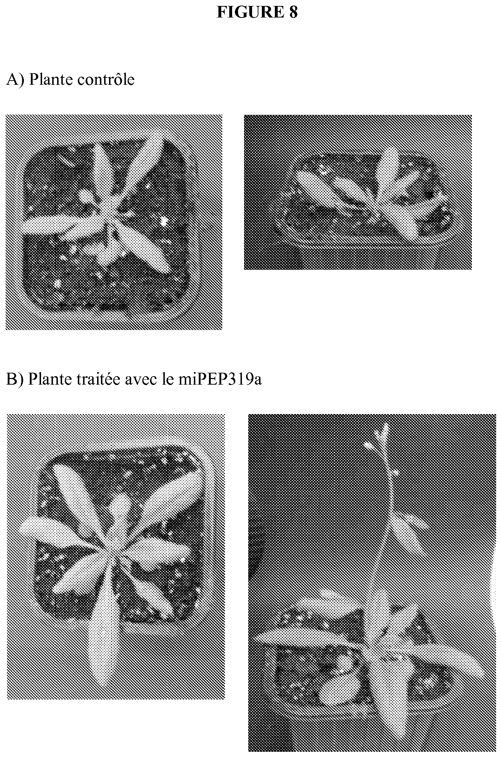

**FIGURE 8****. Effets d'un traitement avec le miPEP319a sur la croissance d'*Arabidopsis thaliana.***
   Les photographies présentent deux plantes (vues de dessus et vues de côté) après 3 semaines de croissance : une plante contrôle arrosée et vaporisée avec de l'eau (A) et une plante arrosée et vaporisée avec une composition de 0,1 µM de peptide synthétique dont la séquence est identique miPEP319a (B). L'arrosage des plantes d'*Arabidopsis thaliana avec* le miPEP319a augmente significativement la croissance de la plante.

### EXEMPLES

### Exemple A1 - Identification et caractérisation du miPEP164a

Pour identifier des miPEPs codés par des pri-miRNAs de plantes, des ORFS ont été recherchés dans des données de RACE-PCR (Xie et al., Plant Physiol, 138:2145-54, 2005), dans lesquelles les séquences de l'extrémité 5' du pri-miR de 50 miRs d'*A*. *thaliana* est disponible.
Des ORFs susceptibles de coder des peptides de 4 à 59 acides aminés ont été identifiés dans chacun des pri-miRs, et notamment un miORF, identifié sur le pri-miR du miR164a, susceptible de coder un peptide miPEP164a.

Pour tester si ce peptide est réellement un miPEP, celui-ci a été synthétisé et a été utilisé pour rechercher une augmentation de l'accumulation du miR164a chez des racines d'*A*. *thaliana* traitées avec le peptide synthétique. Les analyses par Northen blot indiquent que le traitement de la plante avec le peptide miPEP164a entraîne une augmentation de l'accumulation du miR164a (Figure 1).

Par ailleurs, des expériences de croissance ont également été réalisées sur des plantes *A*. *thaliana* et indiquent que l'arrosage des plantes d'*A*. *thaliana* avec le miPEP164a augmente significativement la croissance de la plante (Figure 2).

### Exemple A2 - Matériel et méthodes

### Plantes

Les plantes *A*. *thaliana* Col-0 sont cultivées sur un milieu de base MS complété avec 10 g/l de sucrose.

### Peptides

Les peptides ont été synthétisés par Smartox et dissous dans de l'eau.

### Northern blot

L'analyse par Northern blot a été réalisée selon le protocole décrit dans Lauressergues et al. Plant J, 72(3):512-22, 2012.
Les échantillons biologiques ont été homogénéisés dans un tampon contenant 0,1 M de NaCl, 2% de SDS, 50 mM de Tris-HCl (pH 9), 10 mM de EDTA (PH 8) et 20 mM de mercaptoéthanol, et l'ARN a été extrait 2 fois avec un mélange phénol/chloroforme et précipité à l'éthanol.
L'ARN a été chargé sur gel PAGE 15% et transféré sur une membrane de nylon (HybondNX, Amersham). L'ARN a été hybridé avec une sonde d'oligonucléotides, radioactive marquée à son extrémité, pour détecter l'ARN U6 ou pour le miR164a.
Les hybridations ont été réalisées à 55°c. Les signaux d'hybridation ont été quantifiés en utilisant un phophorimager (Fuji) et normalisés avec le signal de la sonde spécifique de l'ARN U6.

### Test de croissance d'A. thaliana

Les graines d'*Arabidopsis thaliana* ont germé sur GiFi, puis ont été repiquées en terreau et cultivées pendant 3 semaines.
L'arrosage a été réalisé tous les 2-3 jours avec de l'eau pour les plantes contrôles et avec de l'eau contenant 0,1 µM ou 1 µM de peptide synthétique (dont la séquence est identique miPEP164a ou au miPEP165a) pour les plantes tests.

### Exemple B1 - Identification et caractérisation du miPEP319a

Pour identifier des miPEPs codés par des pri-miRNAs de plantes, des ORFS ont été recherchés dans des données de RACE-PCR (Xie et al., Plant Physiol, 138:2145-54, 2005), dans lesquelles les séquences de l'extrémité 5' du pri-miR de 50 miRs d'*A*. *thaliana* est disponible. Des ORFs susceptibles de coder des peptides de 4 à 59 acides aminés ont été identifiés dans chacun des pri-miRs, et notamment un miORF, identifié sur le pri-miR du miR319a, susceptible de coder un peptide miPEP319a.

Pour tester si ce peptide est réellement un miPEP, celui-ci a été synthétisé et a été utilisé pour rechercher une augmentation de l'accumulation du miR319a chez des racines d'*A*. *thaliana* traitées avec le peptide synthétique. Les analyses par qPCR indiquent que la surexpression de AtmiPEP319a induit une augmentation de l'accumulation de Atmir319a (Figure 7).

Par ailleurs, des expériences de croissance ont également été réalisées sur des plantes *A*. *thaliana* et indiquent que l'arrosage et la vaporisation des plantes d'*A*. *thaliana* avec le miPEP319a augmente significativement la croissance de la plante (Figure 8).

### Exemple B2 - Matériel et méthodes

### Plantes

Les plantes *A. thaliana* Col-0 sont cultivées sur un milieu de base MS complété avec 10 g/l de sucrose.

### Peptides

Les peptides ont été synthétisés par Smartox et dissous dans une solution d'eau 40%/ acétonitrile 50%/ acide acétique 10% (v/v/v).

### Transcription inverse des microRNAs

L'ARN a été extrait en utilisant le réactif Tri-Reagent (MRC) selon les instructions du fabricant, à l'exception de la précipitation de l'ARN qui a été réalisée avec 3 volumes d'éthanol. La transcription inverse de l'ARN a été réalisée en utilisant l'amorce spécifique RTprimer tige boucle en combinaison avec des hexamères pour effectuer la transcription inverse de l'ARN de haut poids moléculaire. En bref, 1 µg de RNA a été ajouté à l'amorce tige boucle spécifique (0,2 µM), l'héxamère (500 ng), le tampon RT (1X), l'enzyme SuperScript Reverse transcriptase (SSIII) (une unité), les dNTPs (0,2 mM chacun), le DTT (0,8 mM) dans un mélange réactionnel final de 25 µl. Pour effectuer la transcription inverse, une réaction de transcription inverse pulsée a été réalisée (40 répétitions du cycle suivant : 16°C pendant 2 minutes, 42°C pendant une minute et 50°C pendant une seconde, suivies d'une inactivation finale de la transcription inverse à 85°C pendant 5 minutes).

### Analyses par RT-PCR quantitative (qRT-PCR)

L'ARN total des racines de *M. truncatula* ou des feuilles de tabac a été extrait en utilisant le kit d'extraction RNeasy Plant Mini Kit (Qiagen). La transcription inverse a été réalisée en utilisant la transcriptase inverse SuperScript II (Invitrogen) à partir de 500 ng d'ARN total. Trois répétitions (n=3) ont été réalisées avec deux répétitions techniques chacune. Chaque expérience a été répétée de deux à trois fois. Les amplifications par qPCR ont été réalisées en utilisant un thermocycleur LightCycler 480 System (Roche Diagnostics) selon la méthode décrite dans Lauressergues et al. (Plant J., 72(3):512-22, 2012).

### Constructions plasmidiques

Les fragments d'ADN d'intérêt ont été amplifiés avec la Pfu polymerase (Promega). Les fragments d'ADN ont été clonés pour une surexpression sous le contrôle du promoteur fort constitutif 35S selon la méthode décrite dans Combier et al. (Genes & Dev, 22: 1549-1559, 2008). Le miORF319a a été cloné dans pBIN19 selon la méthode décrite dans Combier et al. (Genes & Dev, 22: 1549-1559, 2008).

### Analyses statistiques

Les valeurs moyennes de l'expression relative des gènes ou de la production de racines latérales ont été analysées en utilisant le test de Student ou le test de Kruskal-Wallis. Les barres d'erreurs représentent l'écart type de la moyenne (SEM, Standard Error of the Mean). Les astérisques indiquent une différence significative (*p*<0,05).

### Test de croissance d'A. thaliana

Les graines d'*Arabidopsis thaliana* ont germé sur GiFi, puis ont été repiquées en terreau et cultivées pendant 3 semaines.

L'arrosage a été réalisé tous les 2-3 jours et la vaporisation a été réalisée tous les jours avec de l'eau pour les plantes contrôles et avec de l'eau contenant 0,1 µM de peptide synthétique (dont la séquence est identique miPEP319a) pour les plantes tests.

## Revendications

1. Utilisation d'un peptide codé par un cadre ouvert de lecture présent sur le transcrit primaire d'un miR (miPEP) introduit par voie externe dans une plante pour en favoriser la croissance, ledit miPEP introduit par voie externe étant un peptide comprenant, ou consistant en, une séquence identique à celle d'un miPEP naturellement présent dans ladite plante,
lequel miPEP naturellement présent est un peptide de 3 à 100 acides aminés, dont la séquence est codée par un cadre ouvert de lecture situé en 5' sur le transcrit primaire d'un miR,
ledit miPEP étant capable de moduler l'accumulation dudit miR dans ladite plante, lequel miR régule l'expression d'au moins un gène impliqué dans le développement des parties végétatives ou reproductrices de la plante, notamment les racines, la tige, les feuilles ou les fleurs,
la somme de la quantité dudit miPEP introduit par voie externe et de celle dudit miPEP naturellement présent étant strictement supérieure à la quantité dudit miPEP naturellement présent.
ledit gène impliqué dans le développement des parties végétatives ou reproductrices de la plante étant choisi parmi le groupe consistant en : *NAC1, NAC4, NAC5, CUC1 et CUC2,* le groupe consistant en : *REVOLUTA, PHABULOSA, PHAVOLUTA, ATHB-8* et *ATHB-15* ou le groupe consistant en : *TCP3* et *TCP4,*
ledit miR étant choisi parmi le groupe consistant en : miR164a, le miR165a ou le miR319a.

2. Utilisation selon la revendication 1, dans laquelle ledit miARN est :
- le miR164a, dans laquelle ledit miR164a possède une séquence nucléotidique consistant en SEQ ID NO : 1,
- le miR165a, , dans laquelle ledit miR165a possède une séquence nucléotidique consistant en SEQ ID NO : 5, ou
- le miR319a, , dans laquelle ledit miR319a possède une séquence nucléotidique consistant en SEQ ID NO : 9.

3. Utilisation selon l'une quelconque des revendications précédentes, dans laquelle ledit miPEP est
- le miPEP164a, en particulier, dans laquelle ledit miPEP164a possède une séquence d'acides aminés consistant en SEQ ID NO : 2,
- le miPEP165a, en particulier, dans laquelle ledit miPEP165a possède une séquence d'acides aminés consistant en SEQ ID NO : 6, ou
- le miPEP319a, en particulier, dans laquelle ledit miPEP319a possède une séquence d'acides aminés consistant en SEQ ID NO : 10.

4. Utilisation selon l'une quelconque des revendications précédentes, dans laquelle ladite plante est une plante crucifère, telle qu'*Arabidopsis thaliana,* une plante solanacée ou une plante légumineuse.

5. Utilisation selon l'une quelconque des revendications 1 à 4, pour favoriser la croissance d'une plante *Arabidopsis thaliana,* dans laquelle le miPEP164a est introduit par voie externe dans ladite plante *Arabidopsis thaliana,* ledit miPEP164a étant également naturellement présent dans ladite plante *Arabidopsis thaliana,* ledit miPEP164a introduit par voie externe étant un peptide dont la séquence comprend ou consiste en une séquence identique à celle dudit miPEP164a naturellement présent, ladite séquence du miPEP164a naturellement présent étant codée par un cadre ouvert de lecture situé en 5' sur le transcrit primaire du miR164a, lequel miR164a régule l'expression d'au moins un gène impliqué dans le développement des parties végétatives ou reproductrices d'*Arabidopsis thaliana,*
la somme de la quantité dudit miPEP164a introduit par voie externe et de celle dudit miPEP164a naturellement présent étant strictement supérieure à la quantité dudit miPEP164a naturellement présent chez ladite plante *Arabidopsis thaliana.*

6. Utilisation selon l'une quelconque des revendications 1 à 4, pour favoriser la croissance d'une plante *Arabidopsis thaliana,* dans laquelle le miPEP165a est introduit par voie externe dans ladite plante *Arabidopsis thaliana,* ledit miPEP165a étant également naturellement présent dans ladite plante *Arabidopsis thaliana,*
ledit miPEP165a introduit par voie externe étant un peptide dont la séquence comprend ou consiste en une séquence identique à celle dudit miPEP165a naturellement présent, ladite séquence du miPEP165a naturellement présent étant codée par un cadre ouvert de lecture situé en 5' sur le transcrit primaire du miR165a, lequel miR165a régule l'expression d'au moins un gène impliqué dans le développement des parties végétatives ou reproductrices d'*Arabidopsis thaliana,*
la somme de la quantité dudit miPEP165a introduit par voie externe et de celle dudit miPEP165a naturellement présent étant strictement supérieure à la quantité dudit miPEP165a naturellement présent chez ladite plante *Arabidopsis thaliana.*

7. Utilisation selon l'une quelconque des revendications 1 à 4, pour favoriser la croissance d'une plante *Arabidopsis thaliana,* dans laquelle le miPEP319a est introduit par voie externe dans ladite plante *Arabidopsis thaliana,* ledit miPEP319a étant également naturellement présent dans ladite plante *Arabidopsis thaliana,* ledit miPEP319a introduit par voie externe étant un peptide dont la séquence comprend ou consiste en une séquence identique à celle dudit miPEP319a naturellement présent, ladite séquence du miPEP319a naturellement présent étant codée par un cadre ouvert de lecture situé en 5' sur le transcrit primaire du miR319a, lequel miR319a régule l'expression d'au moins un gène impliqué dans le développement des parties végétatives ou reproductrices d'*Arabidopsis thaliana,*
la somme de la quantité dudit miPEP319a introduit par voie externe et de celle dudit miPEP319a naturellement présent étant strictement supérieure à la quantité dudit miPEP319a naturellement présent chez ladite plante *Arabidopsis thaliana.*

8. Procédé pour favoriser la croissance d'une plante, comprenant une étape d'introduction d'un miPEP dans une plante par voie externe, ledit miPEP étant également présent naturellement dans ladite plante,
ledit miPEP introduit par voie externe étant un peptide de 3 à 100 acides aminés dont la séquence comprend ou consiste en une séquence identique à celle dudit miPEP naturellement présent, laquelle séquence du miPEP naturellement présent est codée par un cadre ouvert de lecture situé en 5' sur le transcrit primaire d'un miR, ledit miPEP étant capable de moduler l'accumulation dudit miR, lequel miR régule l'expression d'au moins un gène impliqué dans le développement des parties végétatives ou reproductrices de la plante, notamment les racines, la tige, les feuilles ou les fleurs,
la somme de la quantité dudit miPEP introduit par voie externe et de celle dudit miPEP naturellement présent étant strictement supérieure à la quantité dudit miPEP naturellement présent,
ledit gène impliqué dans le développement des parties végétatives ou reproductrices de la plante étant choisi parmi le groupe consistant en : *NAC1, NAC4, NAC5, CUC1 et CUC2,* le groupe consistant en : *REVOLUTA, PHABULOSA, PHAVOLUTA, ATHB-8* et *ATHB-15* ou le groupe consistant en : *TCP3* et *TCP4,*
ledit miR étant choisi parmi le groupe consistant en : miR164a, le miR165a ou le miR319a.

9. Procédé selon la revendication 8, dans lequel ledit miR est :
- le miR164a, ledit miR164a possédant en particulier une séquence nucléotidique consistant en SEQ ID NO : 1,
ledit miPEP étant notamment le miPEP164a, ledit miPEP164a possédant en particulier une séquence d'acides aminés consistant en SEQ ID NO : 2,
- le miR165a, ledit miR165a possédant en particulier une séquence nucléotidique consistant en SEQ ID NO : 5,
ledit miPEP étant notamment le miPEP165a, ledit miPEP165a possédant en particulier une séquence d'acides aminés consistant en SEQ ID NO : 6,
ladite plante étant notamment une plante crucifère, une plante solanacée ou une plante légumineuse, ou
- le miR319a, ledit miR319a possédant en particulier une séquence nucléotidique consistant en SEQ ID NO : 9,
ledit miPEP étant notamment le miPEP319a, ledit miPEP319a possédant en particulier une séquence d'acides aminés consistant en SEQ ID NO : 10,
ladite plante étant notamment une plante crucifère, une plante solanacée ou une plante légumineuse.

10. Procédé selon l'une quelconque des revendications 8 à 9, pour favoriser la croissance d'une plante *Arabidopsis thaliana,* dans lequel :
- le miPEP164a est introduit par voie externe dans ladite plante *Arabidopsis thaliana,* ledit miPEP164a étant également naturellement présent dans ladite plante *Arabidopsis thaliana,*
ledit miPEP164a introduit par voie externe étant un peptide comprenant ou consistant en une séquence identique à celle dudit miPEP164a naturellement présent, lequel miPEP164a naturellement présent est un peptide de 3 à 100 acides aminés dont la séquence est codée par un cadre ouvert de lecture situé en 5' sur le transcrit primaire du miR164a,
ledit miPEP164a étant capable d'augmenter l'accumulation dudit miR164a, lequel miR164a régule l'expression d'au moins un gène impliqué dans le développement des parties végétatives ou reproductrices d'*Arabidopsis thaliana,*
la somme de la quantité dudit miPEP164a introduit par voie externe et de celle dudit miPEP164a naturellement présent étant strictement supérieure à la quantité dudit miPEP164a naturellement présent,
- le miPEP165a est introduit par voie externe dans ladite plante *Arabidopsis thaliana,* ledit miPEP165a étant également naturellement présent dans ladite plante *Arabidopsis thaliana,*
ledit miPEP165a introduit par voie externe étant un peptide comprenant ou consistant en une séquence identique à celle dudit miPEP165a naturellement présent, lequel miPEP165a naturellement présent est un peptide de 3 à 100 acides aminés dont la séquence est codée par un cadre ouvert de lecture situé en 5' sur le transcrit primaire du miR165a,
ledit miPEP165a étant capable d'augmenter l'accumulation dudit miR165a, lequel miR165a régule l'expression d'au moins un gène impliqué dans le développement des parties végétatives ou reproductrices d'*Arabidopsis thaliana,*
la somme de la quantité dudit miPEP165a introduit par voie externe et de celle dudit miPEP165a naturellement présent étant strictement supérieure à la quantité dudit miPEP165a naturellement présent,
ou
- le miPEP319a est introduit par voie externe dans ladite plante *Arabidopsis thaliana,* ledit miPEP319a étant également naturellement présent dans ladite plante *Arabidopsis thaliana,*
ledit miPEP319a introduit par voie externe étant un peptide comprenant ou consistant en une séquence identique à celle dudit miPEP319a naturellement présent, lequel miPEP319a naturellement présent est un peptide de 3 à 100 acides aminés dont la séquence est codée par un cadre ouvert de lecture situé en 5' sur le transcrit primaire du miR319a,
ledit miPEP319a étant capable d'augmenter l'accumulation dudit miR319a, lequel miR319a régule l'expression d'au moins un gène impliqué dans le développement des parties végétatives ou reproductrices d'*Arabidopsis thaliana,*
la somme de la quantité dudit miPEP319a introduit par voie externe et de celle dudit miPEP319a naturellement présent étant strictement supérieure à la quantité dudit miPEP319a naturellement présent.

11. Procédé selon l'une quelconque des revendications 8 à 10, dans lequel ledit miPEP est introduit dans la plante :
- par voie externe de préférence par arrosage, par pulvérisation ou par l'ajout d'un engrais, d'un terreau, d'un substrat de culture ou d'un support en contact avec la plante, ledit miPEP étant notamment administré à la plante sous la forme d'une composition comprenant 10⁻⁹ M à 10⁻⁴ M dudit miPEP, en particulier 10⁻⁹, 10⁻⁸, 10⁻⁷, 10⁻⁶, 10⁻⁵ ou 10⁻⁴ M dudit miPEP,
- par voie externe de préférence par arrosage, par pulvérisation ou par l'ajout d'un engrais, d'un terreau, d'un substrat de culture ou d'un support en contact avec la plante, ledit miPEP étant notamment administré à une graine ou une semence sous la forme d'une composition comprenant 10⁻⁹ M à 10⁻⁴ M dudit miPEP, en particulier 10⁻⁹, 10⁻⁸, 10⁻⁷, 10⁻⁶, 10⁻⁵ ou 10⁻⁴ M dudit miPEP, ou
- par le biais d'un acide nucléique codant ledit miPEP, ledit acide nucléique étant introduit dans la plante.

12. Procédé de production d'une plante transgénique comprenant:
**a)** une étape d'introduction d'un acide nucléique codant un miPEP de 3 à 100 acides aminés dans une plante, mais ledit acide nucléique ne comprenant pas la séquence complète du miR correspondant, ou dans au moins une cellule de ladite plante, dans des conditions permettant l'expression dudit miPEP,
ledit miPEP étant également naturellement présent dans ladite plante, ledit miPEP naturellement présent étant un peptide dont la séquence est codée par un cadre ouvert de lecture situé en 5' sur le transcrit primaire d'un miR, ledit miPEP étant capable de moduler l'accumulation dudit miR dans la plante, lequel miR régule l'expression d'au moins un gène impliqué dans le développement des parties végétatives ou reproductrices de la plante, notamment les racines, la tige, les feuilles ou les fleurs, et
**b)** une étape de culture de la plante, ou d'au moins une cellule de ladite plante, obtenues à l'étape a) dans des conditions permettant l'obtention d'une plante transgénique,
- ledit gène impliqué dans le développement des parties végétatives ou reproductrices de la plante étant choisi parmi le groupe consistant en : *NAC1, NAC4, NAC5, CUC1* et *CUC2,* ledit miARN étant le miR164a, ledit miR164a possédant en particulier une séquence nucléotidique consistant en SEQ ID NO : 1, ou
ledit miPEP étant le miPEP164a, ledit miPEP164a possédant en particulier une séquence d'acides aminés consistant en SEQ ID NO : 2,
- ledit gène impliqué dans le développement des parties végétatives ou reproductrices de la plante étant choisi parmi le groupe consistant en : *REVOLUTA, PHABULOSA, PHAVOLUTA, ATHB-8* et *ATHB-15,*
ledit miARN étant le miR165a, ledit miR165a possédant en particulier une séquence nucléotidique consistant en SEQ ID NO : 5, ou
ledit miPEP étant le miPEP165a, ledit miPEP165a possédant en particulier une séquence d'acides aminés consistant en SEQ ID NO : 6,
ou
- ledit gène impliqué dans le développement des parties végétatives ou reproductrices de la plante étant choisi parmi le groupe consistant en : *TCP3* et *TCP4,*
ledit miARN étant le miR319a, ledit miR319a possédant en particulier une séquence nucléotidique consistant en SEQ ID NO : 9, ou
ledit miPEP étant le miPEP319a, ledit miPEP319a possédant en particulier une séquence d'acides aminés consistant en SEQ ID NO : 10,

13. Plante transgénique telle qu'obtenue par le procédé tel que défini selon la revendication 12.

14. Composition comprenant :
- le miPEP164a en tant que substance active, ledit miPEP164a consistant de préférence en SEQ ID NO : 2, ledit miPEP164a étant à une concentration de 10⁻⁹ M à 10⁻⁴ M, en particulier 10⁻⁹, 10⁻⁸, 10⁻⁷, 10⁻⁶, 10⁻⁵ ou 10⁻⁴ M,
- le miPEP165a en tant que substance active, ledit miPEP165a consistant de préférence en SEQ ID NO : 6, ledit miPEP165a étant à une concentration de 10⁻⁹ ou 10⁻⁴ M, ou
- le miPEP319a en tant que substance active, ledit miPEP319a consistant de préférence en SEQ ID NO : 10, ledit miPEP319a étant à une concentration de 10⁻⁹ M à 10⁻⁴ M, en particulier 10⁻⁹, 10⁻⁸, 10⁻⁷, 10⁻⁶, 10⁻⁵ ou 10⁻⁴ M,
en particulier, ladite composition comprenant de plus un excipient, un diluant ou un solvant, en particulier, ladite composition étant formulée de façon à former un enrobé.

15. Composition comprenant en combinaison une quantité de semences d'une plante et une quantité d'un peptide dont la séquence comprend ou consiste en une séquence identique à celle d'un miPEP naturellement présent chez ladite plante,
ledit peptide ayant une séquence comprenant ou consistant en une séquence identique à celle du miPEP164a, du miPEP165a ou du miPEP319a,
en particulier, ladite composition étant formulée de façon à former une semence enrobée.

## Patentansprüche

1. Verwendung eines Peptids, das durch einen offenen Leserahmen codiert ist, der auf dem primären Transkript einer miR vorhanden ist, (miPEP), und das extern in eine Pflanze eingeführt wird, um das Wachstum zu stimulieren, wobei das genannte miPEP, das extern eingeführt wird, ein Peptid ist, umfassend eine, oder bestehend aus einer, Sequenz, die identisch ist mit jener eines miPEP, das natürlich in der genannten Pflanze vorhanden ist,
wobei das miPEP, das natürlich vorhanden ist, ein Peptid mit 3 bis 100 Aminosäuren ist, deren Sequenz durch einen offenen Leserahmen codiert ist, der 5' auf dem primären Transkript einer miR lokalisiert ist,
wobei das genannte miPEP in der Lage ist, die Akkumulation der miR in der genannten Pflanze zu modulieren, wobei die miR die Expression mindestens eines Gens reguliert, das in die Entwicklung von vegetativen oder reproduktiven Teilen der Pflanze impliziert ist, insbesondere den Wurzeln, dem Stamm, den Blättern oder den Blüten,
wobei die Summe der Menge des genannten miPEP, das extern eingeführt wird, und jener des genannten miPEP, das natürlich vorhanden ist, streng größer ist als die Menge des genannten miPEP, das natürlich vorhanden ist,
wobei das genannte Gen, das in die Entwicklung von vegetativen oder reproduktiven Teilen der Pflanze impliziert ist, ausgewählt ist aus der Gruppe bestehend aus: *NAC1, NAC4, NAC5, CUC1 und CUC2,* die Gruppe bestehend aus: *REVOLUTA, PHABULOSA, PHAVOLUTA, ATHB-8* und *ATHB-15* oder die Gruppe bestehend aus: *TCP3* und *TCP4,*
wobei die genannte miR ausgewählt ist aus der Gruppe bestehend aus: miR164a, miR165a oder miR319a.

2. Verwendung nach Anspruch 1, wobei die genannte miRNA ist:
- die miR164a, wobei die genannte miR164a eine Nucleotidsequenz aufweist, die aus der SEQ ID NO : 1 besteht,
- die miR165a, wobei die genannte miR165a eine Nucleotidsequenz aufweist, die aus der SEQ ID NO : 5 besteht, oder
- die miR319a, wobei die genannte miR319a eine Nucleotidsequenz aufweist, die aus der SEQ ID NO : 9 besteht.

3. Verwendung nach einem der vorhergehenden Ansprüche, wobei das genannte miPEP ist:
- insbesondere das miPEP164a, wobei das genannte miPEP164a eine Aminosäuresequenz aufweist, die aus der SEQ ID NO : 2 besteht,
- insbesondere das miPEP165a, wobei das genannte miPEP165a eine Aminosäuresequenz aufweist, die aus der SEQ ID NO : 6 besteht,
- insbesondere das miPEP319a, wobei das genannte miPEP319a eine Aminosäuresequenz aufweist, die aus der SEQ ID NO : 10 besteht.

4. Verwendung nach einem der vorhergehenden Ansprüche, wobei die genannte Pflanze eine Kruzifere, wie *Arabidopsis thaliana,* eine Solanacea oder eine Leguminose ist.

5. Verwendung nach einem der Ansprüche 1 bis 4, zur Stimulierung des Wachstums einer Pflanze *Arabidopsis thaliana,* wobei das miPEP164a extern in die genannte Pflanze *Arabidopsis thaliana* eingeführt wird, wobei das genannte miPEP164a auch natürlich in der genannten Pflanze *Arabidopsis thaliana* vorhanden ist,
wobei das genannte miPEP164a, das extern eingeführt wird, ein Peptid ist, dessen Sequenz eine Sequenz umfasst oder aus einer Sequenz besteht, die identisch ist mit jener des genannten miPEP164a, das natürlich vorhanden ist, wobei die genannte Sequenz des miPEP164a, das natürlich vorhanden ist, durch einen offenen Leserahmen codiert ist, der 5' auf dem primären Transkript der miR164a lokalisiert ist, wobei die miR164a die Expression mindestens eines Gens reguliert, das in die Entwicklung von vegetativen oder reproduktiven Teilen von *Arabidopsis thaliana* impliziert ist,
wobei die Summe der Menge des genannten miPEP164a, das extern eingeführt wird, und jener des genannten miPEP164a, das natürlich vorhanden ist, streng größer ist als die Menge des genannten miPEP164a, das natürlich in der genannten Pflanze *Arabidopsis thaliana* vorhanden ist.

6. Verwendung nach einem der Ansprüche 1 bis 4, zur Stimulierung des Wachstums einer Pflanze *Arabidopsis thaliana,* wobei das miPEP165a extern in die genannte Pflanze *Arabidopsis thaliana* eingeführt wird, wobei das genannte miPEP165a auch natürlich in der genannten Pflanze *Arabidopsis thaliana* vorhanden ist,
wobei das genannte miPEP165a, das extern eingeführt wird, ein Peptid ist, dessen Sequenz eine Sequenz umfasst oder aus einer Sequenz besteht, die identisch ist mit jener des genannten miPEP165a, das natürlich vorhanden ist, wobei die genannte Sequenz des miPEP165a, das natürlich vorhanden ist, durch einen offenen Leserahmen codiert ist, der 5' auf dem primären Transkript der miR165a lokalisiert ist, wobei die miR165a die Expression mindestens eines Gens reguliert, das in die Entwicklung von vegetativen oder reproduktiven Teilen von *Arabidopsis thaliana* impliziert ist,
wobei die Summe der Menge des genannten miPEP165a, das extern eingeführt wird, und jener des genannten miPEP165a, das natürlich vorhanden ist, streng größer ist als die Menge des genannten miPEP165a, das natürlich in der genannten Pflanze *Arabidopsis thaliana* vorhanden ist.

7. Verwendung nach einem der Ansprüche 1 bis 4, zur Stimulierung des Wachstums einer Pflanze *Arabidopsis thaliana,* wobei das miPEP319a extern in die genannte Pflanze *Arabidopsis thaliana* eingeführt wird, wobei das genannte miPEP319a auch natürlich in der genannten Pflanze *Arabidopsis thaliana* vorhanden ist,
wobei das genannte miPEP319a, das extern eingeführt wird, ein Peptid ist, dessen Sequenz eine Sequenz umfasst oder aus einer Sequenz besteht, die identisch ist mit jener des genannten miPEPR319a, das natürlich vorhanden ist, wobei die genannte Sequenz des miPEP319a, das natürlich vorhanden ist, durch einen offenen Leserahmen codiert ist, der 5' auf dem primären Transkript der miR319a lokalisiert ist, wobei die miR319a die Expression mindestens eines Gens reguliert, das in die Entwicklung von vegetativen oder reproduktiven Teilen von *Arabidopsis thaliana* impliziert ist,
wobei die Summe der Menge des genannten miPEP319a, das extern eingeführt wird, und jener des genannten miPEP319a, das natürlich vorhanden ist, streng größer ist als die Menge des genannten miPEP319a, das natürlich in der genannten Pflanze *Arabidopsis thaliana* vorhanden ist.

8. Verfahren zur Stimulierung des Wachstums einer Pflanze, umfassend einen Schritt des externen Einführens eines miPEP in eine Pflanze, wobei das genannte miPEP auch natürlich in der genannten Pflanze vorhanden ist,
wobei das genannte miPEP, das extern eingeführt wird, ein Peptid mit 3 bis 100 Aminosäuren ist, deren Sequenz eine Sequenz umfasst oder aus einer Sequenz besteht, die identisch ist mit jener des genannten miPEP, das natürlich vorhanden ist, wobei die Sequenz des miPEP, das natürlich vorhanden ist, durch einen offenen Leserahmen codiert wird, der 5' auf dem primären Transkript einer miR lokalisiert ist, wobei das genannte miPEP in der Lage ist, die Akkumulation der miR zu modulieren, wobei die miR die Expression mindestens eines Gens reguliert, das in die Entwicklung von vegetativen oder reproduktiven Teilen der Pflanze impliziert ist, insbesondere den Wurzeln, dem Stamm, den Blättern oder den Blüten,
wobei die Summe der Menge des genannten miPEP, das extern eingeführt wird, und jener des genannten miPEP, das natürlich vorhanden ist, streng größer ist als die Menge des genannten miPEP, das natürlich vorhanden ist,
wobei das genannte Gen, das in die Entwicklung von vegetativen oder reproduktiven Teilen der Pflanze impliziert ist, ausgewählt wird aus der Gruppe bestehend aus: *NAC1, NAC4, NAC5, CUC1 und CUC2,* die Gruppe bestehend aus: *REVOLUTA, PHABULOSA, PHAVOLUTA, ATHB-8* und *ATHB-15* oder die Gruppe bestehend aus: *TCP3* und *TCP4,*
wobei die genannte miR ausgewählt wird aus der Gruppe bestehend aus: miR164a, miR165a oder miR319a.

9. Verfahren nach Anspruch 8, wobei die genannte miR ist:
- die miR164a, wobei die genannte miR164a insbesondere eine Nucleotidsequenz aufweist, die aus der SEQ ID NO : 1 besteht,
wobei das genannte miPEP insbesondere das miPEP164a ist, wobei das genannte miPEP164a insbesondere eine Aminosäuresequenz aufweist, die aus der SEQ ID NO : 2 besteht,
- die miR165a, wobei die genannte miR165a insbesondere eine Nucleotidsequenz aufweist, die aus der SEQ ID NO : 5 besteht,
wobei das genannte miPEP insbesondere das miPEP165a ist, wobei das genannte miPEP165a insbesondere eine Aminosäuresequenz aufweist, die aus der SEQ ID NO : 6 besteht,
wobei die genannte Pflanze insbesondere eine Kruzifere, eine Solanacea oder eine Leguminose ist, oder
- die miR319a, wobei die genannte miR319a insbesondere eine Nucleotidsequenz aufweist, die aus der SEQ ID NO : 9 besteht,
wobei das genannte miPEP insbesondere das miPEP319a ist, das genannte miPEP319a insbesondere eine Aminosäuresequenz aufweist, die aus der SEQ ID NO : 10 besteht,
wobei die genannte Pflanze insbesondere eine Kruzifere, eine Solanacea oder eine Leguminose ist.

10. Verfahren nach einem der Ansprüche 8 bis 9, zur Stimulierung des Wachstums einer Pflanze *Arabidopsis thaliana,* wobei:
- das miPEP164a extern in die genannte Pflanze *Arabidopsis thaliana* eingeführt wird, wobei das genannte miPEP164a auch natürlich in der genannten Pflanze *Arabidopsis thaliana* vorhanden ist,
wobei das genannte miPEP164a, das extern eingeführt wird, ein Peptid ist, umfassend eine oder bestehend aus einer Sequenz, die identisch ist mit jener des genannten miPEP164a, das natürlich vorhanden ist, wobei das miPEP164a, das natürlich vorhanden ist, ein Peptid mit 3 bis 100 Aminosäuren ist, deren Sequenz durch einen offenen Leserahmen codiert wird, der 5' auf dem primären Transkript der miR164a lokalisiert ist,
wobei das genannte miPEP164a in der Lage ist, die Akkumulation der genannten miR164a zu erhöhen, wobei die miR164a die Expression mindestens eines Gens reguliert, das in die Entwicklung von vegetativen oder reproduktiven Teilen von *Arabidopsis thaliana* impliziert ist,
wobei die Summe der Menge des genannten miPEP164a, das extern eingeführt wird, und jener des genannten miPEP164a, das natürlich vorhanden ist, streng größer ist als die Menge des genannten miPEP164a, das natürlich vorhanden ist,
- das miPEP165a extern in die genannte Pflanze *Arabidopsis thaliana* eingeführt wird, wobei das genannte miPEP165a auch natürlich in der genannten Pflanze *Arabidopsis thaliana* vorhanden ist,
wobei das genannte miPEP165a, das extern eingeführt wird, ein Peptid ist, umfassend eine oder bestehend aus einer Sequenz, die identisch ist mit jener des genannten miPEP165a, das natürlich vorhanden ist, wobei das miPEP165a, das natürlich vorhanden ist, ein Peptid mit 3 bis 100 Aminosäuren ist, deren Sequenz durch einen offenen Leserahmen codiert wird, der 5' auf dem primären Transkript der miR165a lokalisiert ist,
wobei das genannte miPEP165a in der Lage ist, die Akkumulation der genannten miR165a zu erhöhen, wobei die miR165a die Expression mindestens eines Gens reguliert, das in die Entwicklung von vegetativen oder reproduktiven Teilen von *Arabidopsis thaliana* impliziert ist,
wobei die Summe der Menge des genannten miPEP165a, das extern eingeführt wird, und jener des genannten miPEP165a, das natürlich vorhanden ist, streng größer ist als die Menge des genannten miPEP165a, das natürlich vorhanden ist, oder
- das miPEP319a extern in die genannte Pflanze *Arabidopsis thaliana* eingeführt wird, wobei das genannte miPEP319a auch natürlich in der genannten Pflanze *Arabidopsis thaliana* vorhanden ist,
wobei das genannte miPEP319a, das extern eingeführt wird, ein Peptid ist, umfassend eine oder bestehend aus einer Sequenz, die identisch ist mit jener des genannten miPEP319a, das natürlich vorhanden ist, wobei das miPEP319a, das natürlich vorhanden ist, ein Peptid mit 3 bis 100 Aminosäuren ist, deren Sequenz durch einen offenen Leserahmen codiert wird, der 5' auf dem primären Transkript der miR319a lokalisiert ist,
wobei das genannte miPEP319a in der Lage ist, die Akkumulation der genannten miR319a zu erhöhen, wobei die miR319a die Expression mindestens eines Gens reguliert, das in die Entwicklung von vegetativen oder reproduktiven Teilen von *Arabidopsis thaliana* impliziert ist,
wobei die Summe der Menge des genannten miPEP319a, das extern eingeführt wird, und jener des genannten miPEP319a, das natürlich vorhanden ist, streng größer ist als die Menge des genannten miPEP319a, das natürlich vorhanden ist.

11. Verfahren nach einem der Ansprüche 8 bis 10, wobei das genannte mPEP in die Pflanze eingeführt wird:
- extern, vorzugsweise durch Berieselung, durch Zerstäubung oder durch Zusatz eines Düngemittels, eines Nährbodens, eines Kultursubstrats oder eines Trägers in Kontakt mit der Pflanze, wobei das genannte miPEP insbesondere der Pflanze in der Form einer Zusammensetzung verabreicht wird, umfassend 10⁻⁹ M bis 10⁻⁴ M des genannten miPEP, insbesondere 10⁻⁹, 10⁻⁸, 10⁻⁷, 10⁻⁶, 10⁻⁵ oder 10⁻⁴ M des genannten miPEP,
- extern, vorzugsweise durch Berieselung, durch Zerstäubung oder durch Zusatz eines Düngemittels, eines Nährbodens, eines Kultursubstrats oder eines Trägers in Kontakt mit der Pflanze, wobei das genannte miPEP insbesondere einem Korn oder einem Samen in der Form einer Zusammensetzung verabreicht wird, umfassend 10⁻⁹ M bis 10⁻⁴ M des genannten miPEP, insbesondere 10⁻⁹, 10⁻⁸, 10⁻⁷, 10⁻⁶, 10⁻⁵ oder 10⁻⁴ M des genannten miPEP, oder
- mit Hilfe einer Nucleinsäure, die für das genannte miPEP codiert, wobei die Nucleinsäure in die Pflanze eingeführt wird.

12. Verfahren zur Herstellung einer transgenen Pflanze, umfassend:
a) einen Schritt des Einführens einer Nucleinsäure, die für ein miPEP mit 3 bis 100 Aminosäuren codiert, in eine Pflanze, wobei die genannte Nucleinsäure jedoch nicht die vollständige Sequenz der entsprechenden miR umfasst, oder in mindestens eine Zelle der genannten Pflanze, unter Bedingungen, welche die Expression des genannten miPEP gestatten,
wobei das genannte miPEP auch natürlich in der genannten Pflanze vorhanden ist, wobei das genannte miPEP, das auch natürlich vorhanden ist, ein Peptid ist, dessen Sequenz durch einen offenen Leserahmen codiert wird, der 5' auf dem primären Transkript einer miR lokalisiert ist, wobei das genannte miPEP in der Lage ist, die Akkumulation der miR in der Pflanze zu modulieren, wobei die miR die Expression mindestens eines Gens reguliert, das in die Entwicklung von vegetativen oder reproduktiven Teilen der Pflanze impliziert ist, insbesondere den Wurzeln, dem Stamm, den Blättern oder den Blüten, und
b) einen Schritt des Kultivierens der Pflanze, oder mindestens einer Zelle der genannten Pflanze, die in dem Schritt a) erhalten werden, unter Bedingungen, die das Erhalten einer transgenen Pflanze gestatten,
- wobei das genannte Gen, das in die Entwicklung von vegetativen oder reproduktiven Teilen der Pflanze impliziert ist, ausgewählt wird aus der Gruppe bestehend aus: *NAC1, NAC4, NAC5, CUC1 und CUC2,*
wobei die genannte miRNA die miR164a ist, wobei die genannte miR164a insbesondere eine Nucleotidsequenz aufweist, die aus der SEQ ID NO : 1 besteht, oder wobei das genannte miPEP das miPEP164a ist, wobei das genannte miPEP164a insbesondere eine Aminosäuresequenz aufweist, die aus der SEQ ID NO : 2 besteht,
- wobei das genannte Gen, das in die Entwicklung von vegetativen oder reproduktiven Teilen der Pflanze impliziert ist, ausgewählt wird aus der Gruppe bestehend aus: *REVOLUTA, PHABULOSA, PHAVOLUTA, ATHB-8* und *ATHB-15,*
wobei die genannte miRNA die miR165a ist, wobei die genannte miR165a insbesondere eine Nucleotidsequenz aufweist, die aus der SEQ ID NO : 5 besteht, oder wobei das genannte miPEP das miPEP165a ist, wobei das genannte miPEP165a insbesondere eine Aminosäuresequenz aufweist, die aus der SEQ ID NO : 6 besteht, oder
- wobei das genannte Gen, das in die Entwicklung von vegetativen oder reproduktiven Teilen der Pflanze impliziert ist, ausgewählt wird aus der Gruppe bestehend aus: *TCP3* und *TCP4,*
wobei die genannte miRNA die miR319a ist, wobei die genannte miR319a insbesondere eine Nucleotidsequenz aufweist, die aus der SEQ ID NO : 9 besteht, oder wobei das genannte miPEP das miPEP319a ist, wobei das genannte miPEP319a insbesondere eine Aminosäuresequenz aufweist, die aus der SEQ ID NO : 10 besteht.

13. Transgene Pflanze, wie erhalten gemäß dem Verfahren wie in Anspruch 12 definiert.

14. Zusammensetzung, umfassend:
- das miPEP164a als aktive Substanz, wobei das genannte miPEP164a vorzugsweise aus der SEQ ID NO : 2 besteht, wobei das genannte miPEP164a in einer Konzentration von 10⁻⁹ M bis 10⁻⁴ M vorliegt, insbesondere 10⁻⁹, 10⁻⁸, 10⁻⁷, 10⁻⁶, 10⁻⁵ oder 10⁻⁴ M,
- das miPEP165a als aktive Substanz, wobei das genannte MiPEP165a vorzugsweise aus der SEQ ID NO : 6 besteht, wobei das genannte miPEP165a in einer Konzentration von 10⁻⁹ M oder 10⁻⁴ M vorliegt, oder
- das miPEP319a als aktive Substanz, wobei das genannte MiPEP319a vorzugsweise aus der SEQ ID NO : 10 besteht, wobei das genannte miPEP319a in einer Konzentration von 10⁻⁹ M bis 10⁻⁴ M vorliegt, insbesondere 10⁻⁹, 10⁻⁸, 10⁻⁷, 10⁻⁶, 10⁻⁵ oder 10⁻⁴ M,
wobei die genannte Zusammensetzung insbesondere außerdem einen Exzipienten, ein Verdünnungsmittel oder ein Lösungsmittel umfasst, wobei die genannte Zusammensetzung insbesondere formuliert ist, um eine Beschichtung zu bilden.

15. Zusammensetzung, umfassend in Kombination eine Menge von Samen einer Pflanze und eine Menge eines Peptids, dessen Sequenz eine Sequenz umfasst oder aus einer Sequenz besteht, die identisch ist mit jener eines miPEP, das natürlich in der genannten Pflanze vorhanden ist,
wobei das genannte Peptid eine Sequenz aufweist, welche eine Sequenz umfasst oder aus einer Sequenz besteht, die identisch ist mit jener von miPEP164a, miPEP165a oder miPEP319a,
wobei die genannte Zusammensetzung insbesondere formuliert ist, um einen beschichteten Samen zu bilden.

## Claims

1. Use of a peptide encoded by an open reading frame present on the primary transcript of a miR (miPEP) miPEP introduced externally into a plant for promoting its growth,
said miPEP introduced externally being a peptide comprising, or consisting of, a sequence identical to that of a miPEP naturally present in said plant,
said miPEP naturally present being a peptide of 3 to 100 amino acids, the sequence of which is encoded by an open reading frame situated at 5' on the primary transcript of a miR,
said miPEP being capable of modulating the accumulation of said miRNA in said plant, which miRNA regulates the expression of at least one gene involved in the development of the vegetative or reproductive parts of the plant, in particular the roots, the stem, the leaves or the flowers,
the sum of the quantity of said miPEP introduced externally and that of said miPEP naturally present being strictly greater than the quantity of said miPEP that is naturally present,
said gene involved in the development of the vegetative or reproductive parts of the plant being selected from the group consisting of: *NAC1, NAC4, NAC5, CUC1* and *CUC2,* the group consisting of: *REVOLUTA, PHABULOSA, PHAVOLUTA, ATHB-8* and *ATHB-15* or the group consisting of: *TCP3* and *TCP4,*
said miR being selected from the group consisting of: miR164a, miR165a or miR319a.

2. Use according to claim 1, in which said miRNA is:
- miR164a, in which said miR164a has a nucleotide sequence consisting of SEQ ID NO: 1,
- miR165a, in which said miR165a has a nucleotide sequence consisting of SEQ ID NO: 5, or
- miR319a, in which said miR319a has a nucleotide sequence consisting of SEQ ID NO: 9.

3. Use according to any one of the preceding claims, in which said miPEP is:
- miPEP164a, in particular in which said miPEP164a has an amino acid sequence consisting of SEQ ID NO: 2,
- miPEP165a, in particular in which said miPEP165a has an amino acid sequence consisting of SEQ ID NO: 6, or
- miPEP319a, in particular in which said miPEP319a has an amino acid sequence consisting of SEQ ID NO: 10.

4. Use according to any one of the preceding claims, in which said plant is a cruciferous plant, such as *Arabidopsis thaliana,* a solanaceous plant or a leguminous plant.

5. Use according to any one of claims 1 to 4, for promoting the growth of an *Arabidopsis thaliana* plant, in which miPEP 164a is introduced externally into said *Arabidopsis thaliana* plant, said miPEP 164a also being naturally present in said *Arabidopsis thaliana* plant,
said miPEP164a introduced externally being a peptide the sequence of which comprises or consists of a sequence identical to that of said miPEP164a naturally present, said sequence of the miPEP164a naturally present being encoded by an open reading frame situated at 5' on the primary transcript of the miR164a, which miR164a regulates the expression of at least one gene involved in the development of the vegetative or reproductive parts of *Arabidopsis thaliana,*
the sum of the quantity of said miPEP164a introduced externally and that of said miPEP164a naturally present being strictly greater than the quantity of said miPEP164a naturally present in said *Arabidopsis thaliana* plant.

6. Use according to any one of claims 1 to 4, for promoting the growth of an *Arabidopsis thaliana* plant, in which miPEP165a is introduced externally into said *Arabidopsis thaliana* plant, said miPEP165a also being present naturally in said *Arabidopsis thaliana* plant,
said miPEP165a introduced externally being a peptide the sequence of which comprises or consists of a sequence identical to that of said miPEP165a naturally present, said sequence of the miPEP165a naturally present being encoded by an open reading frame situated at 5' on the primary transcript of the miR165a, which miR165a regulates the expression of at least one gene involved in the development of the vegetative or reproductive parts of *Arabidopsis thaliana,*
the sum of the quantity of said miPEP165a introduced externally and that of said miPEP165a naturally present being strictly greater than the quantity of said miPEP165a naturally present in said *Arabidopsis thaliana* plant.

7. Use according to any one of claims 1 to 4, for promoting the growth of an *Arabidopsis thaliana* plant, in which miPEP319a is introduced externally into said *Arabidopsis thaliana* plant, said miPEP319a also being naturally present in said *Arabidopsis thaliana* plant,
said miPEP319a introduced externally being a peptide the sequence of which comprises or consists of a sequence identical to that of said miPEP319a naturally present, said sequence of the miPEP319a naturally present being encoded by an open reading frame situated at 5' on the primary transcript of the miR319a, which miR319a regulates the expression of at least one gene involved in the development of the vegetative or reproductive parts of *Arabidopsis thaliana,*
the sum of the quantity of said miPEP319a introduced externally and that of said miPEP319a naturally present being strictly greater than the quantity of said miPEP319a naturally present in said *Arabidopsis thaliana* plant.

8. Method for promoting plant growth, comprising a step of introducing a miPEP into a plant externally, said miPEP also being naturally present in said plant,
said miPEP introduced externally being a peptide of 3 to 100 amino acids the sequence of which comprises or consists of a sequence identical to that of said miPEP naturally present, said sequence of the miPEP naturally present being encoded by an open reading frame situated at 5' on the primary transcript of a miRNA, said miPEP being capable of modulating the accumulation of said miRNA, which miRNA regulates the expression of at least one gene involved in the development of the vegetative or reproductive parts of the plant, in particular the roots, the stem, the leaves or the flowers,
the sum of the quantity of said miPEP introduced externally and that of said miPEP naturally present being strictly greater than the quantity of said miPEP naturally present, said gene involved in the development of the vegetative or reproductive parts of the plant being selected from the group consisting of: *NAC1, NAC4, NAC5, CUC1* and *CUC2,* the group consisting of: *REVOLUTA, PHABULOSA, PHAVOLUTA, ATHB-8* and *ATHB-15* or the group consisting of: *TCP3* and *TCP4,*
said miR being selected from the group consisting of: miR164a, miR165a or miR319a.

9. Method according to claim 8, in which said miR is:
- miR164a, said miR164a having in particular a nucleotide sequence consisting of SEQ ID NO: 1,
said miPEP being in particular miPEP164a, said miPEP164a having in particular an amino acid sequence consisting of SEQ ID NO: 2,
- miR165a, said miR165a having in particular a nucleotide sequence consisting of SEQ ID NO: 5,
said miPEP being in particular miPEP165a, said miPEP165a having in particular an amino acid sequence consisting of SEQ ID NO: 6,
said plant being in particular a cruciferous plant, a solanaceous plant or a leguminous plant, or
- miR319a, said miR319a having in particular a nucleotide sequence consisting of SEQ ID NO: 9,
said miPEP being in particular miPEP319a, said miPEP319a having in particular an amino acid sequence consisting of SEQ ID NO: 10,
said plant being in particular a cruciferous plant, a solanaceous plant or a leguminous plant.

10. Method according to either of claims 8 or 9, for promoting the growth of an *Arabidopsis thaliana* plant, in which:
- miPEP164a is introduced externally into said *Arabidopsis thaliana* plant, said miPEP 164a also being naturally present in said *Arabidopsis thaliana* plant,
said miPEP164a introduced externally being a peptide comprising or consisting of a sequence identical to that of said miPEP164a naturally present, which miPEP164a naturally present is a peptide of from 3 to 100 amino acids the sequence of which is encoded by an open reading frame situated at 5' on the primary transcript of the miR164a,
said miPEP164a being capable of increasing the accumulation of said miR164a, which miR164a regulates the expression of at least one gene involved in the development of the vegetative or reproductive parts of *Arabidopsis thaliana,*
the sum of the quantity of said miPEP164a introduced externally and that of said miPEP164a naturally present being strictly greater than the quantity of said miPEP164a naturally present,
- miPEP165a is introduced externally into said *Arabidopsis thaliana* plant, said miPEP165a also being naturally present in said *Arabidopsis thaliana* plant,
said miPEP165a introduced externally being a peptide comprising or consisting of a sequence identical to that of said miPEP165a naturally present, which miPEP165a naturally present is a peptide of from 3 to 100 amino acids the sequence of which is encoded by an open reading frame situated at 5' on the primary transcript of the miR165a,
said miPEP165a being capable of increasing the accumulation of said miR165a, which miR165a regulates the expression of at least one gene involved in the development of the vegetative or reproductive parts of *Arabidopsis thaliana,*
the sum of the quantity of said miPEP165a introduced externally and that of said miPEP165a naturally present being strictly greater than the quantity of said miPEP165a naturally present,
or
- miPEP319a is introduced externally into said *Arabidopsis thaliana* plant, said miPEP319a also being naturally present in said *Arabidopsis thaliana* plant,
said miPEP319a introduced externally being a peptide comprising or consisting of a sequence identical to that of said miPEP319a naturally present, which miPEP319a naturally present is a peptide of from 3 to 100 amino acids the sequence of which is encoded by an open reading frame situated at 5' on the primary transcript of the miR319a,
said miPEP319a being capable of increasing the accumulation of said miR319a, which miR319a regulates the expression of at least one gene involved in the development of the vegetative or reproductive parts of *Arabidopsis thaliana,*
the sum of the quantity of said miPEP319a introduced externally and that of said miPEP319a naturally present being strictly greater than the quantity of said miPEP319a naturally present.

11. Method according to any one of claims 8 to 10, in which said miPEP is introduced into the plant:
- externally, preferably by watering, by spraying or by adding a fertilizer, a compost, a culture substrate or a support in contact with the plant, said miPEP being in particular administered to the plant in the form of a composition comprising from 10⁻⁹ M to 10⁻⁴ M of said miPEP, in particular 10⁻⁹, 10⁻⁸, 10⁻⁷, 10⁻⁶, 10⁻⁵ or 10⁻⁴ M of said miPEP,
- externally, preferably by watering, by spraying or by adding a fertilizer, a compost, a culture substrate or a support in contact with the plant, said miPEP being in particular administered to a grain or seed in the form of a composition comprising from 10⁻⁹ M to 10⁻⁴ M of said miPEP, in particular 10⁻⁹, 10⁻⁸, 10⁻⁷, 10⁻⁶, 10⁻⁵ or 10⁻⁴ M of said miPEP, or
- by means of a nucleic acid encoding said miPEP, said nucleic acid being introduced into the plant.

12. Method for producing a transgenic plant comprising:
**a)** a step of introducing a nucleic acid encoding a miPEP of from 3 to 100 amino acids into a plant, but said nucleic acid does not comprise the complete sequence of the corresponding miR, or into at least one cell of said plant, under conditions allowing the expression of said miPEP,
said miPEP also being naturally present in said plant, said miPEP naturally present being a peptide the sequence of which is encoded by an open reading frame situated at 5' on the primary transcript of a miRNA, said miPEP being capable of modulating the accumulation of said miRNA in the plant, which miRNA regulates the expression of at least one gene involved in the development of the vegetative or reproductive parts of the plant, in particular the roots, the stem, the leaves or the flowers, and
**b)** a step of growing the plant, or at least one cell of said plant, obtained in step a) under conditions allowing a transgenic plant to be obtained,
- said gene involved in the development of the vegetative or reproductive parts of the plant being selected from the group consisting of: *NAC1, NAC4, NAC5, CUC1* and *CUC2,*
said miRNA being in particular miR164a, said miR164a having in particular a nucleotide sequence consisting of SEQ ID NO: 1, or
said miPEP being in particular miPEP164a, said miPEP164a having in particular an amino acid sequence consisting of SEQ ID NO: 2,
- said gene involved in the development of the vegetative or reproductive parts of the plant being selected from the group consisting of: *REVOLUTA, PHABULOSA, PHAVOLUTA, ATHB-8* and *ATHB-15,*
said miRNA being in particular miR165a, said miR165a having in particular a nucleotide sequence consisting of SEQ ID NO: 5, or
said miPEP being in particular miPEP165a, said miPEP165a having in particular an amino acid sequence consisting of SEQ ID NO: 6, or
- said gene involved in the development of the vegetative or reproductive parts of the plant being selected from the group consisting of: *TCP3* and *TCP4,*
said miRNA being in particular miR319a, said miR319a having in particular a nucleotide sequence consisting of SEQ ID NO: 9, or
said miPEP being in particular miPEP319a, said miPEP319a having in particular an amino acid sequence consisting of SEQ ID NO: 10.

13. Transgenic plant as obtained by the method as defined according to claim 12.

14. Composition comprising:
- miPEP164a as active ingredient, said miPEP164a preferably consisting of SEQ ID NO: 2, said miPEP164a being in particular at a concentration of from 10⁻⁹M to 10⁻⁴M, in particular 10⁻⁹, 10⁻⁸, 10⁻⁷, 10⁻⁶, 10⁻⁵ or 10⁻⁴ M,
- miPEP165a as active ingredient, said miPEP165a preferably consisting of SEQ ID NO: 6, said miPEP165a being in particular at a concentration of from 10⁻⁹M to 10⁻⁴M, or
- miPEP319a as active ingredient, said miPEP319a preferably consisting of SEQ ID NO: 10, said miPEP319a being in particular at a concentration of from 10⁻⁹ M to 10⁻⁴ M, in particular 10⁻⁹, 10⁻⁸, 10⁻⁷, 10⁻⁶, 10⁻⁵ or 10⁻⁴ M,
in particular, said composition further comprising an excipient, a diluent or a solvent,
in particular, said composition being formulated so as to form a coating.

15. Composition comprising in combination an quantity of seeds from a plant and an quantity of a peptide the sequence of which comprises or consists of a sequence identical to that of a miPEP naturally present in said plant,
said peptide having a sequence comprising or consisting of a sequence identical to that of miPEP164a, miPEP165a or miPEP319a,
in particular, said composition being formulated so as to form a coated seed.
